(19) Europäisches Patentamt
European Patent Office
Office européen des brevets

(11) EP 0 945 138 A1

(12) **EUROPEAN PATENT APPLICATION**

(43) Date of publication:
**29.09.1999 Bulletin 1999/39**

(51) Int. Cl.$^6$: **A61K 47/48**

(21) Application number: **97121308.7**

(22) Date of filing: **04.12.1997**

(84) Designated Contracting States:
**AT BE CH DE DK ES FI FR GB GR IE IT LI LU MC NL PT SE**
Designated Extension States:
**AL LT LV MK RO SI**

(71) Applicants:
- **Université Louis Pasteur de Strasbourg**
  **F-67000 Strasbourg (FR)**
- **BOEHRINGER INGELHEIM INTERNATIONAL GmbH**
  **55218 Ingelheim am Rhein (DE)**

(72) Inventors:
- **Behr, Jean Paul**
  **F-67100 Strasbourg (FR)**
- **Blessing, Thomas**
  **A-1110 Wien (AT)**
- **Wagner, Ernst, Dr.**
  **A-2103 Langenzersdorf (AT)**

(74) Representative:
**Laudien, Dieter, Dr.**
**Boehringer Ingelheim GmbH**
**Abteilung Patente,**
**Postfach 200**
**55216 Ingelheim (DE)**

# (54) Transfection particles

(57) Transfection particles for delivery of nucleic acid into higher eucaryotic cells *in vitro* and *in vivo* comprises one or more nucleic acid molecules condensed by organic cationic molecules. The discrete and stable particles are obtained by complexing the nucleic acid molecules with identical or different organic cationic precursor molecules without crosslinking nucleic acid molecules, and covalently linking the precursor molecules to each other on the nucleic acid template. For specific cellular targeting, the particles may carry targeting molecules, e.g. sugars. Preferred cationic precursor molecules are lipophilic detergents that are linked to form lipids. The particles contain preferably only one nucleic acid molecule which makes them useful for gene therapy and for delivery of large DNA molecules.

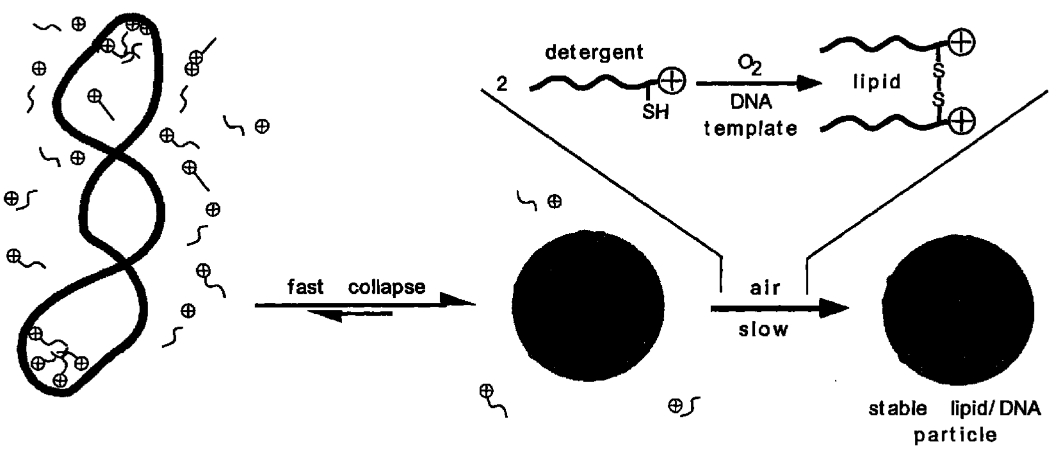


Fig. 1



Printed by Xerox (UK) Business Services
2.16.7/3.6

## Description

Field of the invention

The present invention relates to the field of nucleic acid delivery into higher eucaryotic cells, in particular for applications in gene therapy. The invention provides new transfection particles that are useful for transfecting higher eucaryotic cells *in vitro* and in vivo.

Background of the invention

A critical step of modern recombinant nucleic acid technology is the efficient transfection of cells *in vitro* and in vivo with foreign genetic material.

Commonly, recombinant viruses are used for efficiently carrying foreign genetic material into cells *in vitro* and in vivo (Mulligan, 1993). Recombinant viruses are often cell specific which may be of advantage or disadvantage depending on the individual experiment. In addition to the foreign material they also carry their own genetic material and are highly prone to mutational changes during replication. In organisms with immune systems a further disadvantage of viruses in vivo is a possible unwanted immune response triggered by virus particles such as capsid antigens. Last but not least, even today the preparation of recombinant viruses for gene transfer is an expensive and laborious task.

Virus-free nucleotide particles are also available for transfecting cells. For this purpose, complexes of nucleic acid molecules, counterion-condensed by cationic polymers (Tang and Szoka, 1997) or cationic lipids (Labatmoleur et al., 1996), are employed for genetic transfer. These polymer and lipid condensed nucleic acid molecules are efficient at transfecting cells in culture. Since these counterion-condensed nucleotides generally lead to multimolecular and polydisperse aggregates they are very poor at diffusing within a tissue or escaping blood vessels.

Cationic detergents too have been shown to condense DNA into discrete particles (Behr, 1986) which, interestingly, can consist of only a single nucleic acid molecule (Melnikov et al., 1995). However, detergents have a much higher water solubility than lipids and their fast release into the environment induces DNA-decondensation and detergent-related cytotoxicity. As a consequence, this class of amphiphiles is unable to transfect cells *per se* (Behr, 1993).

Because of the importance of gene delivery for modern biological and medical research and therapy, for example gene therapy, there is a growing demand for stable, inexpensive and convenient transfection systems that are useful for *in vitro* and in vivo applications.

Summary of the invention

It was the object of the present invention to provide a novel transfection system for delivering nucleic acid into higher eucaryotic cells that circumvents the disadvantages of the cationic transfection systems of the state of the art.

The present invention provides particles for transfecting higher eucaryotic cells with nucleic acid molecules *in vitro* and *in vivo* comprising one or more nucleic acid molecules condensed by organic cationic molecules, said particles being obtained by complexing the nucleic acid molecules with identical or different organic cationic precursor molecules without crosslinking nucleic acid molecules, and covalently linking the precursor molecules to each other on the nucleic acid template.

The particles of the invention are, due to the use of small cationic precursor molecules instead of larger cationic molecules, essentially free of aggregated nucleic acid. Furthermore, the particles are stabilized by the dimeric or oligomeric cationic molecules formed by covalent linkage of the precursor molecules.

In the following, unless otherwise defined, for simplicity, the term DNA is used for both DNA and RNA.

The stability of the particles should be reversible in order to make the DNA available for its desired biological effect in the cell. To this end, in a preferred embodiment of the invention, the linkage between the cationic molecules is reversible under the conditions that the particles are faced after entry into the cell. The break of the linkage may be triggered by the slightly acidic environment and/or the reducing conditions and/or enzymatic machinery of the cellular compartments, e.g. the endosomes.

The cationic molecules that condense the DNA are obtained by covalently linking the cationic precursor molecules.

Cationic precursor molecules according to the invention are defined in that they are building blocks for the preparation of the cationic molecules that keep the DNA condensed.

The general concept of the invention requires one or more at least bifunctional precursor molecules that are on the one hand cationic in order to complex nucleic acid molecules essentially without aggregating or crosslinking it, and on the other hand capable of covalently linking to identical or different cationic precursor molecules of the particle structure for stabilizing that particle.

A function for covalently linking to identical or different cationic precursor molecules for stabilizing a novel transfection particle can be characterized by different and complementary functions or identical functions. An example of an identical function is a thiol that is capable of providing a disulfide bridge between identical or different precursor cationic molecules. Depending on the number of functions for linking on a single cationic precursor molecule the cationic molecules can be dimers or oligomers of different or identical precursor molecule. Therefore, the invention also relates to transfection particles characterized in that the cationic molecules are prepared from demised or oligomerized identical and/or different cationic precursor molecules.

The cationic function for condensing nucleic acid molecules is generally a basic function that acquires an additional hydrogen under physiological conditions and therefore becomes positively charged. An example of such a cationic function is an amino function or a derivative thereof. Alternatively, the cationic function is a quarternaryzed amine which does not require protonation.

In a preferred embodiment of the invention, the precursor molecules are cationic detergent molecules that satisfy the above-defined requirements.

In this preferred embodiment, the invention is based on the consideration of the basics of supramolecular chemistry and takes advantage of the essential features of two types of DNA delivery agents (cationic surfactants on the one hand and cationic lipids on the other hand) towards the formation of stable transfection particles reminiscent of viruses. Exemplified for this preferred embodiment, the principle of the invention is schematically depicted in Fig. 1: anionic DNA molecules are condensed individually with an appropriately designed cationic detergent and the resulting neutral particles are 'frozen' by chemically converting the detergent (the cationic precursor molecule) on the template DNA into a lipid via air-induced dimerization.

The detergent molecules of the invention comprise a cationic polar part, an unpolar part and a reactive function for linking to other detergent molecules. When two or more detergent molecules are linked to each other the detergent character of the original precursor molecules is reduced and they are then defined as lipids because of their lipophilic character and structural similarity to lipids. At this point the reader should be reminded that transfection particles prepared by adding plain lipids to nucleic acid molecules result in large multimolecular and polydisperse aggregates (Labatmoleur et al., 1996) and are not part of the claimed invention.

In a further aspect, the present invention is related to transfection particles characterized in that the cationic molecules are lipid molecules prepared from detergent molecules linked to one or more identical or different detergent molecules in the presence of nucleic acid molecules.

In a preferred embodiment the invention relates to transfection particles characterized in that the cationic molecules are prepared by dimerization or oligomerization of detergent molecules. Obviously, the detergents can be identical or different in structure.

In general, cationic detergent molecules for use as cationic precursor molecules can be characterized by their distinct functional features.

In a further aspect of the invention transfection particles are characterized in that the cationic detergent molecules comprise:

a) at least one function for binding to one or more other detergent molecules,

b) at least one lipophilic residue,

c) a non-toxic recipient backbone,

d) a cationic group for binding to DNA.

Functional residues for binding to precursor molecules are, for example but not limited to, thiol residues that react to provide disulfide bridges (-S-S-), acid hydrazides and aldehydes that provide hydrazones (-C=N-N-), amines and aldehydes that provide Schiff bases (imines,-C=N-), and amines that react with ethylene residues that are suitably substituted (e.g. halides) to provide enamines (-C=C-N-).

In this respect, the transfection particles are preferably characterized in that the function of the cationic detergent molecules for binding to other detergent molecules is selected from dimerizable and polymerizable functions, such as thiols, acid hydrazides, aldehydes, amines, and ethylene residues that are suitably substituted to provide enamines.

Preferably, a transfection particle is characterized in that the lipophilic residue of the detergent molecule is selected from lipophilic amides, esters or ethers. The lipophilic functions of the cationic molecules are critical when using detergents as precursor molecules since the lipophilic residues determine the critical micelle concentration of the detergent precursor that is suitable to provide discrete particles at a desired concentration. The higher the desired DNA concentration, the higher the c.m.c. of the detergent needs to be.

For binding to nucleic acid molecules the preferred functional residues of cationic molecules and especially for detergent molecules are amines or other basic and cationic derivatives thereof. Suitable amines and derivative are discussed further below.

Preferably, a transfection particle of the invention is characterized in that the function for binding to nucleic acid molecules is selected from an amine or derivative thereof.

The amino functions suitable for the invention depend on their ability to provide positive charges for binding nucleic acid molecules reversibly by charge dependent ionic forces. The ability of amines and derivatives to be positively charged under physiological conditions depends on the structural proximity of the amine. Some structures with close and strong electron drawing substituents may therefore not be basic enough under physiological conditions. Preferably, amino function of cationic molecules of the invention are strongly basic. One very basic and physiologically acceptable amino function is guanidine which is especially useful for the cationic molecules of the invention. A further example for a suitable amino function is amidine. Both of these functions exhibit a strong interaction with phosphate diester groups.

Preferably, a transfection particle of the invention is characterized in that the function for binding to nucleic acid molecules is guanidine.

The requirements for the recipient backbone for detergent molecules according to the invention are obvious, convenient to prepare and non-toxic to cells.

In a preferred embodiment, the invention relates to transfection particles characterized in that the cationic precursor molecules correspond to general formula I

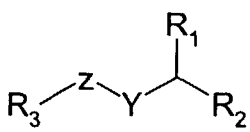

(I)

wherein

$R_1$ denotes ($C_1$-$C_{10}$-alkylene)-SH, wherein the alkylene radical may represent a straight chained or branched hydrocarbon;

$R_2$ denotes $-NR_4R_5$, $-NHR_4R_5^+$, $-N(R_4)_2R_5^+$, $-C(=NR_4)NR_5R_6$, $-C(=X)-C_1-C_{10}$-alkylene, wherein the alkylene radical may represent a straight chained or branched hydrocarbon and may be substituted by up to four dialkyl amino groups or a thiomonosaccharide; $R_3$ denotes $C_5$-$C_{30}$-alkyl, straight chained or branched and optionally substituted preferably with one or more halogen atom(s) or dialkyl amino group(s), or

$C_5$-$C_{30}$-alkenyl, straight chained or branched having up to ten C=C-double bonds and is optionally substituted preferably with one or more halogen atom(s) or dialkyl amino group(s), or

$C_5$-$C_{30}$-alkinyl, straight chained or branched having up to ten C≡C-triple bonds and is optionally substituted preferably with one or more halogen atom(s) or dialkyl amino group(s), or

$C_6$-$C_{10}$-aryl optionally substituted, or

$C_7$-$C_{16}$-aralkyl optionally substituted, or a

$C_5$-$C_{30}$-alkyl-chain interrupted by up to 10 amino groups $-NR_4-$ and having optionally an amino-group which is optionally substituted by an amino acid;

$R_4$, $R_5$ and $R_6$ denote independently from each other hydrogen or $C_1$-$C_4$-alkyl;

X denotes O or S;

Y denotes C=O or C=S and

Z denotes O, S or $-NR_4-$.

In a more preferred embodiment, the invention relates to transfection particles characterized in that the cationic precursor molecules correspond to general formula I wherein

$R_1$ denotes ($C_1$-$C_6$-alkylene)-SH, wherein the alkylene radical may represent a straight chained or branched hydrocarbon;

$R_2$ denotes $-NR_4R_5$, $-NHR_4R_5^+$, $-N(R_4)_2R_5^+$, $-C(=NR_4)NR_5R_6$, -C(=X)-$C_1$-$C_4$-alkylene, wherein the alkylene radical may represent a straight chained or branched hydrocarbon and may be substituted by up to four amino radicals -$NR_4R_5$ or a monosaccharide;

$R_3$ denotes $C_5$-$C_{20}$-alkyl, straight chained or branched and optionally substituted preferably with F, Cl, Br or -$NR_4R_5$, or

$C_5$-$C_{20}$-alkenyl, straight chained or branched having up to five C=C-double bonds and is optionally substituted preferably with F, Cl, Br or -$NR_4R_5$, or

$C_5$-$C_{20}$-alkinyl, straight chained or branched having up to five C≡C-triple bonds and is optionally substituted preferably with F, Cl, Br or -$NR_4R_5$, or

$C_6$-$C_{10}$-aryl optionally substituted preferably with $C_1$-$C_4$-alkyl, F, Cl, Br or -$NR_4R_5$, or

$C_7$-$C_{14}$-aralkyl optionally substituted preferably with $C_1$-$C_4$-alkyl, F, Cl, Br or -$NR_4R_5$, or

a $C_5$-$C_{20}$-alkyl chain interrupted by up to 10 amino groups -$NR_4$- and having optionally an amino-group which is optionally substituted by an amino acid;

$R_4$, $R_5$ and $R_6$ denote independently from each other hydrogen or $C_{1-}C_{4-}$alkyl;

X denotes O or S;

Y denotes C=O or C=S and

Z denotes O, S or -$NR_4$-.

In an even more preferred embodiment, the invention relates to transfection particles characterized in that the cationic precursor molecules according to the general structure described above are substituted as follows:

$R_1$ denotes ($C_1$-$C_3$-alkylene)-SH, wherein the alkylene radical may represent a straight chained or branched hydrocarbon;

$R_2$ denotes $-NR_4R_5$, $-NHR_4R_5^+$, $-N(R_4)_2R_5^+$, $-C(=NR_4)NR_5R_6$, -C(=X)-$C_1$-$C_4$-alkyl, wherein the alkyl radical may represent a straight chained or branched hydrocarbon and may be substituted by up to four amino radicals -$NR_4R_5$, or a $C_6$-monosaccharide;

$R_3$ $C_5$-$C_{12}$-alkyl, straight chained or branched and optionally substituted preferably with F, Cl, Br or -$NH_2$, or a

$C_5$-$C_{15}$-alkyl chain interrupted by up to 7 amino groups -$NR_4$- and having optionally a -amino-group which is optionally substituted by the amino acid cysteine;

$R_4$, $R_5$ and $R_6$ denote independently from each other hydrogen or methyl, ethyl, propyl, iso-propyl, n-butyl, iso-butyl or tert.-butyl;

X denotes O or S;

Y denotes C=O or C=S and

Z denotes O, S or -$NR_4$-.

In a most preferred embodiment, the invention relates to transfection particles characterized in that the cationic precursor molecules according to the general structure described above are substituted as follows:

$R_1$ denotes $-CH_2-SH$;

$R_2$ denotes $-NH_2$, $-NH_3^+$, $-C(=NH^+{}_2)NH_2$, $-C(=O)-C_1-C_4$-alkyl straight chained or branched and optionally substituted with F, Cl, Br or $-NH_2$
or an ornithine radical or a S-galactosyl radical;

$R_3$ denotes a $C_6$-$C_{15}$-alkyl radical straight chained or branched and optionally substituted preferably with F, Cl, Br or $-NH_2$,

Y denotes C=O;

Z denotes O or -NH-.

Preferred are transfection particles characterized in that $R_1$ is $C_1$-$C_4$-alkylene-SH. More preferred are transfection particles characterized in that $R_1$ is a methylenethiol.
Preferred are transfection particles characterized in that $R_2$ is an amine. More preferred are transfection particles characterized in that $R_2$ is a guanidine. Also more preferred are transfection particles characterized in that $R_2$ is an ornithine.
Preferred are transfection particles characterized in that $R_3$ is a decyl radical.
Preferred are transfection particles characterized in that Y is a carbonyl.
Also preferred are transfection particles characterized in that Z is an amine.
Most preferred are transfection particles characterized in that $R_1$ is a methylenethiol, $R_2$ is a guanidine, $R_3$ is a decane, Y is a carbonyl, Z is an amine, and pharmaceutically acceptable salts thereof.
Especially preferred are transfection particles characterized in that the cationic molecule is N-decyl-2-guanidinium-cysteine.
Also most preferred are transfection particles characterized in that $R_1$ is a methylenethiol, $R_2$ is an ornithine, $R_3$ represents a straight chained decyl radical, Y is a carbonyl, Z is an amine, and pharmaceutically acceptable salts thereof.
Also especially preferred are transfection particles characterized in that the cationic molecule is N-decyl-2-ornithinyl-cysteine.
$C_1$-$C_6$-alkyl generally represents a straight-chained or branched hydrocarbon radical having 1 to 6 carbon atoms which may optionally be substituted by one or several halogen atoms - preferably fluorine - which may be identical to one another or different. The following radicals may be mentioned by way of example:

methyl, ethyl, propyl, 1-methylethyl (isopropyl), butyl, 1-methylpropyl, 2-methylpropyl, 1,1-dimethylethyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and1-ethyl-2methyl-propyl.

The same definition applies accordingly to alkanediyl radicals.
$C_5$-$C_{30}$-alkyl refers specifically to a straight-chained or branched hydrocarbon radical having 5 to 30 carbon atoms - for example pentyl, hexyl, heptyl, octyl, nonyl, decyl, undecyl, dodecyl, tridecyl, tetradecyl, pentadecyl, hexadecyl, dodecadecyl, nonadecyl, eicosyl, henicosyl, docosyl and triacontyl.
Unless otherwise stated, lower alkyl groups having 1 to 5 carbon atoms such as methyl, ethyl, n-propyl, isopropyl, n-butyl, tert.-butyl, n-pentyl or iso-pentyl are preferred. The same definition applies to alkandiyl radicals.
Alkenyl in general represents a straight-chained or branched hydrocarbon radical having 3 to 20 carbon atoms and one or more double bonds, preferably one double bond, which may optionally be substituted by one or several halogen atoms - preferably fluorine - which may be identical to another or different. Examples include:

2-propenyl (allyl), 2-butenyl, 3-butenyl, 1-methyl-2-propenyl, 2-methyl-2-propenyl, 2-pentenyl, 3-pentenyl, 4-pentenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-2-propenyl, 1-ethyl-2-propenyl, 2-hexenyl, 3-hexenyl, 4-hexenyl, 5-hexenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 3-methyl-4-pentenyl, 4-methyl-4-pentenyl, 1,1-dimethyl-2-butenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 1,3-dimethyl-3-butenyl, 2,2-dimethyl-3-butenyl, 2,3-dimethyl-2-butenyl, 2,3-dimethyl-3-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl and 1-ethyl-2-methyl-2-propenyl.

Alkinyl in general represents a straight chained or branched hydrocarbon radical having 3 to 20 carbon atoms and one or more triple or one or more triple and double bonds.

2-propynyl (propargyl), 2-butynyl, 3-butynyl, 1-methyl-2-propynyl, 2-pentynyl, 3-pentynyl, 4-pentynyl, 1-methyl-2-butynyl, 1-methyl-3-butynyl, 2-methyl-3-butynyl, 1,1-dimethyl-2-propynyl, 1-ethyl-2-propynyl, 2-hexynyl, 3-hexynyl, 4-hexynyl, 5-hexynyl, 1-methyl-2-pentynyl, 4-methyl-2-pentynyl, 1-methyl-3-pentynyl, 2-methyl-3-pentynyl, 1-methyl-4-pentynyl, 3-methyl-4-pentynyl, 1,1-dimethyl-2-butynyl, 1,1-dimethyl-3-butynyl, 1,2-dimethyl-3-butynyl, 1,3-dimethyl-2-butynyl, 1,3-dimethyl-3-butynyl, 2,2-dimethyl-3-butynyl, 2,3-dimethyl-2-butynyl, 2,3-dimethyl-3-butynyl, 1-ethyl-2-butynyl, 1-ethyl-3-butynyl, 2-ethyl-3-butynyl and 1-ethyl-1-methyl-2-propynyl.

Monosaccharides in the meaning of the present invention represent $C_5$- and prerferably $C_6$- monosaccharides and are for example selected from the following group:

galactose, lactose, glucose, arabinose , fructose, sorbose, xylose, ribose, mannose each of them in their D- or L-form.

Aryl in general represents an aromatic radical having 6 to 10 carbon atoms - also in combinations, it being possible for the aromatic ring to be substituted by one or more lower alkyl group(s), alkoxy group(s), nitro group(s), amino group(s) and/or one or more halogen atom(s) - which are identical to one another or different. The preferred aryl group is mentioned - unless otherwise stated - phenyl.

Aralkyl in general represents an aryl radical having 7 to 14 carbon atoms which is bonded via an alkylene chain, it being possible for the aromatic ring to be substituted by one more lower alkyl group(s), alkoxy group(s), nitro group(s), amino group(s) and/or one or more halogen atom(s) - which are identical to one another or different. Aralkyl radicals having 1 to 6 carbon atoms in the aliphatic partial structure and 6 to 10 carbon atoms in the aromatic part are preferred. Preferred aralkyl radicals which may be mentioned - unless otherwise stated - are: benzyl, phenethyl and phenylpropyl.

Alkoxy in general represents an O-alkyl radical wherein alkyl is as defined above. Preferred alkoxy radicals have lower alkyl groups as defined above.

Alkyl or dialkylamino represents an amino radical substituted by one or two alkyl groups which are identical to one another or different. Preferred alkyl or dialkylamino substituents are amino radicals having one or more lower alkyl groups.

In a further embodiment of the invention, the organic cationic precursor molecule is free of a lipophilic residue. Suitable molecules of this type are able to bind to DNA molecules without crosslinking or aggregating them. To fulfill this requirement, they should be small enough to adhere to a single DNA molecule only, without binding to one or more other DNA molecules. In addition, the cationic molecules contain functions that allow them to be covalently linked to other identical or different cationic precursor molecules. The requirements for the functional groups are the same as described for the detergent precursor molecules. Cationic molecules can be tested for their suitability for the present invention by contacting them with DNA and physically examining the obtained complexes for particle size, e.g. by electron microscopy, laser light scattering or atomic force microscopy, before and after covalent linkage. Suitable cationic molecules show no aggregate formation before or after linkage; on the other hand, they must exhibit particle formation after linkage, optionally also before linkage.

Cationic non-lipophilic precursor molecules may be selected from the group comprising molecules with a backbone carrying positively charged groups. The backbone is not critical, it may be any organic molecule that carries a positive charge, either in the main chain or in a side chain. As an example, the backbone is a peptide, the positive charges are provided by positively charged amino acids, e.g. lysine, arginine, ornithine. Suitable positively charged groups may be the same as defined above for the detergent molecules. Provided that they fulfill the above requirements, the organic cationic precursor molecules may be those that are disclosed in WO 93/0783 for complexing DNA.

The functional groups for covalent linking may be provided by the backbone itself, e.g. if the molecule is a peptide and has cysteine residues, or they may be obtained by linking them to the backbone.

Alkyl or dialkylamino represents an amino radical substituted by one or two alkyl groups which are identical to one another or different. Preferred alkyl or dialkylamino substituents are amino radicals having one or mere lower alkyl groups.

In a preferred embodiment transfection particles are characterized in that the cationic precursor molecule is an amine or polyamine.

In a particular preferred embodiment transfection particles are characterized in that the cationic precursor molecule is spermine-N1, N12-bis-cysteineamide ($SC_2$).

The aforementioned spermine derivative fulfills all requirements necessary for practicing the invention. Primary and secondary amines provide cationic functions under physiological conditions and two thiol groups allow for dimerization or even oligomerization without crosslinking or aggregating DNA.

The cationic precursor molecule spermine-N1,N12-bis-cysteineamide ($SC_2$) can be prepared from commercially available reagents. In the experiments of the invention the biophysical parameters of transfection particles prepared with $SC_2$ were determined and it was shown that, as opposed to particles prepared with spermine, they are stable.

The $SC_2$ molecule is composed of a spermine molecule linked via an amide bond to two cysteine molecules having two free thiol functions. The choice of these two molecules is rather critical. Spermine as well as other polyamines, e.g. putrescine, spermidine, is implicated in maintaining viral DNA in condensed form. Various *in vitro* studies have shown that polyamines compact DNA, and under optimal conditions spermine is capable of condensing DNA into toroid structures of 0.1 μm diameter. Nevertheless these toroids are, although relatively stable over time, sensitive to the saline concentration of the medium. As a matter of fact, at a concentration of 150 mM NaCl (which is the ionic strength of the cell culture medium) spermine decomplexes from the DNA which results in the loss of the toroid structures. Therefore particles of DNA complexed with spermine cannot be utilized for transfection. In order to stabilize the particle structure of the DNA complex, two groups carrying a free thiol function each are grafted at each end of the spermine. Under certain optimized conditions, the SC2 molecules present in the minor groove of the DNA, which serves as a template, will bind to each other via a disulfide bond, thus providing a backbone for the DNA which is capable of stabilizing the toroid conformation.

The invention is by no means limited to one type of cationic precursor molecules within the transfection particle. The transfection particles may be prepared by covalently linking two or more different cationic precursor molecules. It is obvious that for cationic precursor molecules to be linked to different cationic precursor molecules, they must be complementary and therefore different from the other reaction partner with regard to their functional groups. Cationic precursor molecules of the invention may also provide a structural variety beside the functional variety for linking other cationic precursor molecules. For specific applications, an expert of the art may find it useful to design transfection particles that satisfy specific requirements by combining structurally different cationic precursor molecules.

Transfection particles have to be stable for the time needed to reach their destination, e.g. target cell or tissue. At the target site, e.g. within a target cell, it is of course necessary to make the nucleic acids of the transfection particles available to that cell.

Material foreign to cells is generally taken up by endocytosis and passes through a series of compartments, inter alia endosomes. Within endosomes, the foreign material is confronted with conditions such as a more acidic pH of about 6 and a great variety of enzymes, especially hydrolases. Transfection particles according to the invention can be taken up by cells and consequently be transferred to endosomal compartments. Within the intracellular compartments, the transfection particles of the invention encounter conditions that cause their disassembly, in particular by cleavage of the dimeric or oligomeric cationic molecules, thereby releasing the nucleic acids for decondensation. Typical break of cationic molecules is caused by the reduction of disulfide bridges; examples for acid labile linkages are hydrazones, Schiff bases (imines), enamines, acetal or ketal functions. It should be noted that the invention is by no means limited to the bonds mentioned above for endosomal cleavage and that an expert of the art is aware of alternative bonds that fulfill the requirements for cationic molecules and are cleavable in the endosomes or other intracellular compartments.

Therefore, in a preferred embodiment of the invention, the transfection particles are characterized by a linkage between the cationic molecules which is degradable under the conditions of the intracellular compartments.

The novel transfection particles are useful for transfecting cells with nucleic acid molecules *in vitro* as well as *in vivo.* The novel transfection particles are especially useful for transfecting higher eucaryotic cells *in vitro* and *in vivo.*

For efficient delivery of nucleic acid molecules it may be desirable to provide specific cellular targeting functions. This is important for *in vivo* applications to target genes into specific cells, tissues or organs. Transfection efficiency may further be increased by the enhanced endocytotic uptake of the particles into the cells once they are bound to the target cells.

In a further aspect, the invention therefore relates to transfection particles characterized in that they additionally carry one or more functions for specific cellular targeting and/or facilitating endocytosis.

Efficient cellular uptake of the transfection particles can be achieved by ligands that bind to cellular structures on the cell surface and/or trigger internalization of the bound structures.

The cellular targeting functions are, for example, either protein ligands or sugar residues. Examples for ligands are immunoglobulins and fragments or derivatives thereof, lectins, adhesion molecules, cytokines or chemokines, as well as small organic structures, e.g. sugar residues, that recognize specific cellular surface structures and are capable of specific targeting and/or internalization. Well-established examples for targeting functions are transferrin, folic acid, RGD peptide, and a great number of monoclonal and polyclonal antibody. Any ligand that is capable of binding to the

target cell and internalizing the particles into the cell may be used, examples for suitable ligands that may be used in the present invention are given in WO 93/0783.

Therefore, in a preferred embodiment the invention relates to transfection particles characterized in that the targeting and/or endocytotic function is a cellular protein ligand.

In a further embodiment the invention relates to transfection particles according to the invention characterized in that the targeting and/or endocytotic function is a sugar residue.

In addition to targeting ligands, the particles of the invention may contain a function that enables them to cross cellular membranes, either by membrane fusion, disruption or pore formation (in the following, this function is termed endosomolytic function). In case the cationic precursor molecules are detergents, they have *per se* a membrane destabilizing/endosomolytic function. If the covalent linkage is bioreversible and therefore broken down in the cellular environment, the detergent molecules can exert their endosomolytic function. Alternatively or in addition to the detergent's membrane destabilizing function, this function may be provided by endosomolytic agents. Examples for suitable endosomolytic agents that may be used in the present invention, are disclosed in WO 93/0783.

Preferred examples for endosomolytic agents are inactivated adenovirus particles, membrane active peptides (i.e. fusogenic or lytic peptides, examples of which have been disclosed in WO 93/0783 and by Plank et al., 1994; Mechtler and Wagner, 1997; Gottschalk et al., 1996; the DNA binding membrane active peptide disclosed by Wyman et al., 1997; the amphipathic cationic peptide gramicidin disclosed by Legendre and Szoka, 1993 and Legendre and Supersaxo, 1995), and pH sensitive surfactants such as N-alkyl-imidazoles which are disclosed by Wilson et al; 1987 Hughes et al., 1996 and by the references therein.

The targeting and/or endosomolytic functions of the transfection particles of the invention may be covalently linked to few, many or all cationic molecules of the invention and may be identical or different targeting functions within a single transfection particle. The presence of targeting/endosomolytic function, both in terms of type and amount, depends on the specific application, in particular on the cell type to be reached and may be adjusted to the particular requirements. In general, the percentage of cationic molecules that carry such functions may be about 1 to 80 %, preferably about 10 to 50 %.

An example of a precursor molecule with a targeting function that is capable of binding covalently to precursor molecules of the cationic molecules of the invention is a lipophilic galactosyl cysteine (Remy et al., 1995) (see example 7). Although this lipophilic galactosyl cysteine does not provide *per se* the functionality required for complexing nucleic acid molecules it is capable of covalently binding to cationic precursor molecules to provide cationic molecules (heterodimers) that are part of the transfection particles of the invention. Galactosyl residues are known to be targeting functions specifically recognized by liver cells.

The targeting/endosomolytic function may also be attached to a DNA binding molecule that is present in addition to the olimerisable precursor molecule. This molecule may be a oligocation like oligolysine or a polymer like polyethyleneimine without dimerizable functions. These targeting/endosomolytyc conjugates may be added to the other complex partners of the transfection particle before or during complex formation.

The ligand/endosomolytic function may be incorporated into the transfection particles in the following ways:

a) providing the cationic precursor molecules and/or non-oligomerizable small DNA binding molecules that are equipped with a targeting/endosomolytic function before or during complex formation;

b) attaching the targeting/endosomolytic function to the cationic precursor molecules and/or or non-oligomerizable small DNA binding molecules after complex formation before or during covalently linking the precursor molecules;

c) attaching the targeting/endosomolytic function after covalently linking the precursor molecules.

In the case of b) and c) the precursor molecules or non-oligomerizable molecules are equipped with a reactive group that allows for binding the targeting/endosomolytic function. Reactive group means chemically reactive, e.g. activated esters, disulfides, or allowing for biochemical interaction, e.g. streptavidin/biotin binding (in this case the molecule is equipped with streptavidin or biotin to react). The reactive group must be selected such that it does not interfere with the process of covalently linking the precursor molecules.

The embodiment a) has the advantage that the molecules carrying the targeting/endosomolytic function can be prepared in advance in purified form in larger amounts and are readily available as well-defined reagents. This embodiment is in particular applied for the endosomolytic function when it is desirable to have this function within the transfection particle.

Embodiments b) and c) are preferred for large ligands, like protein targeting ligands, or endosomolytic agents like adenoviruses because these ligands/agents may interfere with condensation of DNA complexes to small particles. It is an advantage of these embodiments that it they guarantee that the function is located at the surface of the particle. Embodiment c) is additionally advantageous if the ligand is sensitive to the conditions used for condensing and/or covalently linking of precursor molecules.

In a further embodiment, the present invention is directed to transfection particles in which the targeting function is provided by a sugar residue.

In a preferred embodiment, the sugar is galactose.

In a further embodiment, the endosomolytic function is provided by a fusogenic peptide.

Methods for attaching agents providing targeting/endosomolytic functions, e.g. small organic molecules such as sugars or larger structures such as proteins or entire viruses, to the cationic molecules are available to the expert.

Binding by chemical methods can be effected in the manner which is already known for the coupling of peptides and, if necessary the individual components may be provided with linker substances before the coupling reaction (this measure is necessary if there is no functional group available which is suitable for the coupling, e.g. a mercapto or alcohol group). The linker substances are bifunctional compounds which are reacted first with functional groups of the individual components, after which the modified individual components are coupled.

Coupling may be carried out by means of

a) disulphide bridges, which can be cleaved again under reducing conditions (e.g. when using succinimidyl-pyridyldithiopropionate);

b) compounds which are substantially stable under biological conditions (e.g. thioethers by reacting maleimido linkers with sulfhydryl groups of the linker bound to the second component);

c) bridges which are unstable under biological conditions, e.g. ester bonds, or acetal or ketal bonds which are unstable under slightly acidic conditions;

d) reactive groups like isothiocyanate, activated esters;

e) reactive coupling of aldehydes with amino groups.

In a preferred embodiment, the nucleic acid of the transfection particles is DNA.

The DNA may be linear or circular, single-stranded or double stranded. Concerning the size of the DNA, the invention allows for a wide range between small oligonucleotides containing approximately 20 nucleotides and very large molecules like artificial chromosomes of ca. 100 to 2000 kb.

In a preferred embodiment, the particles are monomolecular with respect to the DNA, which means that they contain a single DNA molecule. This embodiment is particularly useful for the delivery of DNA *in vivo* where it is desirable to apply small particles in order to overcome the size limitations of the physiological barriers.

Furthermore, the availability of monomolecular discrete stable particles is advantageous for the delivery of large DNA molecules *in vitro* and *in vivo* because such particles are more readily taken up by cells than large aggregates.

The particles may, however, contain more than one DNA molecules, in particular when smaller DNA molecules like oligonucleotides are applied.

The particles of the invention are generally spherical in structure, their diameter may vary from approximately 25 nm (in case of plasmid DNA) to several hundred nm. It has been found that the volume of the formed particles is directly proportional to the length of DNA. This strongly suggests that the condensation of the DNA molecules is monomolecular.

In a further aspect, the invention comprises the use of cationic molecules and/or cationic precursor molecules for preparing novel nucleotide transfection particles.

In a further aspect, the invention comprises a method for preparing transfection particles, characterized in that cationic precursor molecules are added to nucleic acid molecules in a suitable buffer, allowed to form complexes with the nucleic acid and allowed to covalently link to identical or different cationic precursor molecules on the nucleic acid template.

In a first step of the method, a DNA solution which contains one type of DNA molecules or a mixture of different molecules in a buffer is mixed with a solution of precursor molecules, optionally with different precursor molecule solutions.

Starting from the DNA concentration that is desired in the final transfection formulation and a given ratio of positive and negative charges, which depends on the specific application, the amount and the physical characteristics (solubility, c.m.c., etc.) of the precursor molecules are determined. Specific applications may be defined by the administration route, e.g. the systemic or the intravenous route, for which the transfection particles should not be positively charged in order to avoid undesired reactions with blood components, or by the cells to be targeted, which may require considering the preference for specific for binding charged particles. For selecting a suitable detergent as a precursor molecule, its critical micelle (c.m.c.) concentration, which is known for a given detergent or may be determined by means of a Langmuir balance, is important. For the concept of the invention, optimal results are obtained if the concentration of the detergent is below the c.m.c.

The components are incubated under conditions that allow for complex formation; usually the complexes can form at room temperature during a short period of time, i.e. between several minutes and few hours. After mixing the complex partners, the precursor molecules are covalently linked, which may, dependent on the rate of complex formation, occur immediately after mixing or after the required longer period of time. Depending on the reactive groups available for linkage, linkage may occur spontaneously upon contact of the precursor molecules when bound to DNA. Alternatively, linkage may need to be mediated by a an additional agent, such as oxygen. The conditions need to be such that they do not affect the DNA molecule, therefore most precursor molecules that are preferably used in the present invention are dimerizable or oligomerizable simply by air. The suitable reaction conditions and the time course of the reaction steps can be determined by carrying out routine experiments in which the parameters are varied and the physical characteristics of the (intermediate) reaction products (complexes and compacted particles) are monitored, e.g. particle size measurement by laser light scattering.

In case targeting/endosomolytic functions are present or are to be provided on the precursor molecules or on additional, non-dimerizable cationic molecules, the method, e.g. in terms of incubation period for complex formation or introducing additional steps for modification of the cationic molecules, may be adapted.

Suitable buffers for preparing the novel transfection particles are any buffers that allow complex formation and/or are compatible with the conditions for covalent linkage between individual cationic precursor molecules and for attaching the optional targeting/endosomolytic function. Other parameters such as the optimum pH value, the incubation time, the temperature, and the concentrations of nucleic acids and cationic precursor molecules and optionally molecules for targeting and/or endocytosis depend on each individual embodiment. These parameters may be optimized in routine serial experiments without undue burden for any the expert because the parameters are limited, for example to aqueous systems and to moderate conditions that do not alter the nucleic acid molecules. Furthermore, the Examples describe such parameters which may be easily modified. For example, suitable parameters for oxidizing cysteine precursor molecules in the presence of nucleic acid molecules that comprise a decane hydrophilic function and a guanidine cationic function to provide disulfide bridges are a stock buffer solution of 15 mM TrisHCl, pH 8.4, saturated with oxygen from three vacuum/oxygen atmosphere cycles, 90 μM (final concentration) cationic precursor molecules added before the addition of 60 μM (final concentration) plasmid DNA.

A preferred use for the novel transfection particles of the invention is *in vivo* delivery of nucleic acid into mammalian cells for therapeutic applications.

In this case the nucleic acid is therapeutically active, e.g. an antisense oligonucleotide a plasmid encoding a therapeutically active protein. There are no limitations in terms of DNA sequence, any sequence that is useful in therapy, e.g. somatic gene therapy or immunotherapy, may be used. Non-limiting examples are given in WO 93/0783.

Therefore, in a further aspect, the invention relates to pharmaceutical compositions for gene delivery comprising transfection particles containing a pharmaceutically effective amount of a nucleic acid.

Such pharmaceutical compositions may comprise transfection particles prepared freshly before administration, or off-the-shelf preparations which may be stored as a cooled liquid, in frozen form or as a lyophilisate, and which may contain addtional additives such as stabilizers, cryoprotectives, etc. Suitable additives are known in the art; reference is made to Remington's Pharmaceutical Sciences, 1980, for methods of formulating pharmaceutical compositions.

Apart from *in vivo* DNA delivery, the particles of the invention are particularly useful for introducing large DNA molecules, e.g. artificial chromosomes into mammalian cells, which may be important for regulated long-term gene expression.

For *in vivo* and *in vitro* transfection a kit of parts for the preparation of transfection particles with the desired nucleic acid molecules can provide great convenience. Such a kit of parts may contain, for example, one or more types of cationic precursor molecules that may be used in different combinations to optimize their properties, one or more molecules for cell specific targeting/endosomolytic function, suitable buffers, and other reagents or mechanical devices that are useful for preparing, purifying or applying the novel transfection particles *in vitro* and *in vivo.*

In the following, the invention is exemplified for the cationic precursor molecule N-decyl-2-guanidinium-cysteine:

The detergent molecule was designed de novo (Example 1). Due to the preference for mild chemistry and the use of endogeneous-like compounds thiol was chosen as a dimerizable function, and cysteine was chosen as the non-toxic recipient backbone for constructing the polyfunctional molecule. It can be expected that once inside the endosomal compartment both lipophilic cysteine ester or amide vectors will be enzymatically cleaved back to cysteine and an aliphatic alcohol or amine with concomitant DNA decondensation and possible endosome lysis.

Clean monomolecular DNA collapse is generally expected only for detergent concentrations below the critical micelle concentration (c.m.c.). Several n-alkyl esters of cysteine were synthesized by DMAP-catalyzed dicyclohexylcarbodiimide (DCC) dehydration and their c.m.c.'s were determined with a Langmuir balance (20 mM MES buffer, pH 6), providing the following results (n-alkyl chain length / c.m.c.): dodecane / 15 μM; undecane / 80 μM; decane / 320 μM; octane /> 2000 μM. Typical working plasmid concentrations for gene delivery experiments being in the 50 μM range, the decane chain is a safe choice. The weakly basic nature of the cysteine ester primary amine function (pKa= 6.8) is insufficient for simultaneous DNA binding (which requires a protonated detergent) and reasonable thiol oxidation rates in slightly basic medium. The amine function was therefore converted into a highly basic guanidine which is known to interact strongly with DNA (C10 -CG+, Fig. 2).

Oxidative detergent dimerization was expected to be prone to a template effect of DNA (Fig. 1). Fast intra-complex reaction is a key aspect to formation of small and stable particles since extensive detergent dimerization into lipid prior to DNA binding would bring the system back to a classical cationic lipid/DNA complex formation/aggregation process. Enhanced air oxidation rate was indeed observed in the presence of DNA (Fig. 3).

Below the c.m.c., the major attractive force is that of anionic DNA towards isolated cationic amphiphile molecules. Prior to oxidation, complexes should therefore be close to electroneutrality even when the detergent is slightly in excess. Therefore plasmid DNA (60 μM in 15 mM TrisHCl pH 8.4) was mixed with C10 -CG+ (to 90 μM) and left in aerobic conditions for 24 h. During the instantaneous complex formation step, the typical UV absorption spectrum of DNA centered at 260 nm was increased by 15%; no further change (once corrected for disulfide absorption) and no turbidity was observed during oxidation. The CD spectrum of the complexes was typical of B-DNA, ruling out the occurrence of a liquid crystalline Y-DNA phase in the complexes. It remained time-independent over a period of 24 h. The most stringent test of stability, however, was that performed in physiologically relevant ionic concentrations, where cationic lipid/DNA complexes are known to aggregate3, 9. Remarkably, the UV spectrum of (C10 -CG+)2/DNA complexes remained unchanged after 3 days in 150 mM NaCl. Spectroscopy thus gave indirect evidence for the formation of small and stable amphiphile/DNA complexes.

Transmission electron microscopy (TEM) of an unfiltered solution of these complexes displayed a surprisingly homogeneous population of nearly spherical objects (71% monomers, n=109) with a few larger structures (Fig. 4a). Moreover, the larger structures were clearly dimers (Fig. 4b, d) or oligomers which may have arisen incidentally and which displayed no tendency to merge into more intimately fused aggregates as cationic lipids do3. Raising stepwise the detergent concentrations above the c.m.c. of C10 -CG+ progressively led to less clearcut UV and TEM results due to time-dependent aggregation (data not shown), in agreement with the hypotheses underlying the concept. At the highest magnification (Fig. 4c) the particle size was measured to be 23±4 nm. A rough calculation using typical DNA and amphiphile molecular dimensions led to a sphere of 28 nm diameter when adding the volumes of a 5.5 Kbp plasmid to that of 5 500 lipid molecules. TEM thus provides strong evidence for monomolecular DNA collapse.

In a series of experiments, the reversibility of complex formation as well as the stability of complexed DNA towards degradation were assessed by electrophoresis (Fig. 5). While C10 -CG+ was unable to retard DNA migration, except for concentrations well above the c.m.c. where presumably precipitation occurs (not shown), the oxidized (C10 -CG+)2/DNA particles did not migrate and were stable during electrophoresis (Fig. 5, lane 2). Yet the cationic lipid/DNA particles could be destroyed either with excess sodium dodecyl sulfate detergent (SDS, lane 3) or by converting the lipid back to the original C10 -CG+ detergent with excess reducing dithiothreitol (DTT, lane 4). Serum nucleases present in cell culture medium rapidly degrade supercoiled plasmid DNA (lanes 1 and 8) into a smear of fragments with many nicks (lane 5). The lipid/DNA particles, however, are physically stable in this medium (lane 6) and protect to a large extent the nucleic acid from degradation, as the plasmid is essentially converted into a slower migrating circular relaxed form (revealed after SDS treatment, lane 7).

Being devoid of excess cationic surface charge, these particles are not expected to bind efficiently to cell-surface polyanions (Labatmoleur, 1996, Mislick, 1996). As a consequence they should not transfect cells.

Preliminary experiments performed with a luciferase reporter gene and BNL CL.2 murine hepatocytes indeed confirmed this. However cationic particles formed with a 3-fold excess detergent led to reporter gene expression, confirming that, once in the cell, the fate of the complexes was that of a non-viral vector.

Brief description of the figures

Fig. 1: Schematic representation of cationic detergent-induced monomolecular collapse of plasmid DNA

Fig. 2: Synthesis of a lipophilic guanidyl-cysteine amide detergent

Fig. 3: Oxydation rate of cysteine detergent $C_{10}$ -$\mathbf{C}^{G+}$ into cystine lipid ($C_{10}$ -$\mathbf{C}^{G+}$)$_2$ occurs faster in the presence of template DNA

Fig. 4: Transmission electron microscopy of an unfiltered solution of $(C_{10}\text{-}\mathbf{C}^{G+})_2$ cationic lipid / DNA particles

Fig. 5: Physical and chemical stability of the $(C_{10}\text{-}\mathbf{C}^{G+})_2$ / DNA complexes (agarose gel electrophoresis)

Fig. 6: Nanosizing DNA particles condensed with $C_{10}\text{-}C^{G+}$ Measurement of particle size

A: Phage lambda DNA
B: Phage T4 DNA
C: CMV-luc DNA

Fig. 7A,B: Nanosizing DNA particles condensed with $C_{10}\text{-}C^{G+}$ Proof for monomolecular DNA condensation

Fig. 8: Transfection of lipophilic guanidyl cysteine amide ($C_{10}\text{-}C^{G+}$)/DNA complexes into the cell line BNLCL2

Fig. 9: Synthesis of a lipophilic alcohol cysteine ester detergent

Fig. 10: Determination of the critical micelle concentration (c.m.c.) of the lipophilic alcohol cysteine ester detergents with and without DNA. c.m.c. of a detergent with C8, C10, C11 and C12 cysteine.

Fig. 11: Determination of the critical micelle concentration (c.m.c.) of the lipophilic alcohol cysteine ester detergents with and without DNA A: c.m.c. of C10 cysteine detergent with and without DNA B: c.m.c. of C11 cysteine detergent with and without DNA)

Fig. 12: Oxydation of C10 cysteine detergent with and without DNA

Fig. 13: Synthesis of a lipophilic alcohol guanidyl-cysteine ester detergent

Fig. 14: Synthesis of a lipophilic ornithyl-cysteine amide detergent

Fig. 15: Synthesis of a lipophilic alcohol galactosyl-cysteine ester detergent

Fig. 16: UV spectroscopy of spermine-N1,N12-bis-cysteineamide or spermine particles

Fig. 17: Agarose gel electrophoresis. Stability of particles formed either with spermine or with spermine-N1,N12-bis-cysteineamide

Abbreviations

Ac = $CH_3$ - C(O)
DCC = dicyclohexylcarbodiimide
DMAP = dimethylaminopyridine
DCU = dicyclohexylurea
DDT = dithiothreitol
DMEM = Dulbeccos Modified Minimum Essential Medium
EDC = N-(3-Dimethylaminopropyl)-N- ethyl-carbodiimide hydrochloride
Et = $C_2H_5$
Ether = diethylether
HEPES = hydroxyethylpiperazino ethane sulfonic acid
Me = Methyl
MES = Morpholinoethane sulfonic acid
MS = mass spectrometry
MES = PEI = polyethylenimine
BocON = 2-tert-butoxycarbonyl-oxyimino)-2-phenylacetonitrile
μM bp = micromolar concentration of DNA calculated per base pair (MW = 660)
$C_{10}\text{-}\mathbf{C}^{G+}$ = Lipophilic guanidyl-cysteine amide
*TA* = Tris-acetate

THF = tetrahydrofuran
TLC = thin layer chromatography

Example 1

a) Synthesis of a lipophilic guanidyl-cysteine amide detergent

The Synthesis of the lipophilic compound was carried out according to the scheme as depicted in Fig. 2.

Step 1

Manufacture of N-*tert*-butoxycarbonyl-pyrazole-1-carboxamidine

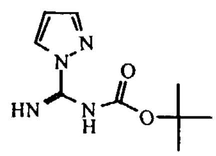

To a solution of di-tert-butyl dicarbonate (7.43 g, 33 mmol) in 100 ml dichloromethane 11.3 ml of N,N-diisopropylethylamine (66 mmol) and 4.40 g of 1H-pyrazole-1-carboxamidine hydrochloride (30 mmol) were added. The solution was agitated for 3 h at room temperature and then evaporated to dryness. The obtained white solid was dissolved in 200 ml ethylacetate, the organic phase was washed with 5 % (w/w) sodium hydrogen carbonate, dried over magnesium sulfate and finally evaporated to dryness. The residue was dissolved in boiling hexane. The solution was allowed to stand at room temperature over night and then at 4°C for 24 hours. The crystals thus formed were recovered by filtration and washed in cold hexane (0°C). After drying, 5.28 g of white crystals were obtained (25 mmol yield=84 %).

$C_9H_{14}N_4O_2$; 210,24 g/mol

TLC: Rf (20% AcOEt / Hexane) = 0,3.
TLC on reverse phase: Rf (60% $CH_3CN$ / 30% $H_2O$ / 10% MeOH) = 0,5.
NMR$^1$H ($CDCl_3$); $\delta$ ppm: 1,56 (s, 9H, C(**CH**$_3$)$_3$); 6,41 (dd, $J_1$=3 Hz, $J_2$=1,5 Hz, 1H, CH-**CH**=CH); 7,50-7,70 (brs, 1H, =**NH**); 7,69 (d, J=1,1 Hz, 1H, N=**CH**); 8,46 (d, J=3 Hz, 1H, N-**CH**); 9,01-9,20 (brs, 1H, **NH**Boc).(In this and in the other NMR spectra, the hydrogen atoms assigned to the signal are in bold print.)
MS (FAB+), m/z : 211,1 [$MH^+$]; 155,0 [$MH^+$ - *t*-Butyl].

Step 2

Manufacture of N,N'-bis-*tert*-butoxycarbonyl-pyrazole-1-carboxamidine

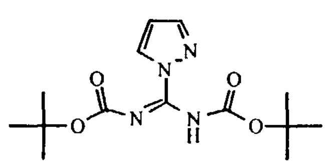

To a suspension of sodium hydride (1.60 g, 40 mmol) in 70 ml tetrahydrofurane at 0°C 2.10 g N-*tert*-butoxycarbonyl-pyrazole-1-carboxamidine (10 mmol) were added. The suspension was intensely agitated for 1 h at 0°C. Then 4.5 g di-tert-butyl dicarbonate (20 mmol) were added and placed at room temperature and then gradually heated to the reflux. After one night under reflux the reaction medium was cooled to 0°C; 2.3 ml acetic acid were added and the solution was agitated during 15 min. The organic phase was washed with 60 ml 5 % (w/w) sodium hydrogen carbonate, 60 ml of a saturated NaCl solution, dried over magnesium sulfate and then evaporated to dryness. The obtained residue was purified by silica chromatography (gradient 5 to 15 % ethylacetate in hexane) to obtain 2.48 g of a white solid (8.0 mmol, yield=80 %).

$C_{14}H_{22}N_4O_4$; 310,36 g/mol

TLC : Rf (20% AcOEt / Hexane) = 0,3.
TLC on reverse phase: Rf (60% $CH_3CN$ / 30% $H_2O$ / 10% MeOH) = 0,4.
NMR$^1$H ($CDCl_3$); δ ppm: 1,52 (s, 9H, C(**CH$_3$**)$_3$); 1,58 (s, 9H, C(**CH$_3$**)$_3$); 6,43-6,46 (m, 1H, CH-**CH**=CH); 7,64-7,66 (m, 1H, N=**CH**); 8,33 (d, J=3 Hz, 1H, N-**CH**); 8,93 (brs, 1H, **NH**Boc).
MS (FAB+), m/z: 311,1 [MH$^+$]; 255,0 [MH$^+$ - *t*-Butyl]; 211,0 [MH$^+$ - Boc]; 199,0 [MH$^+$ - 2*t*-Butyl]; 155,0 [MH$^+$ - Boc - *t*-Butyl].

Step 3

Manufacture of N,S-bis-*tert*-butoxycarbonylcysteine-decylamide

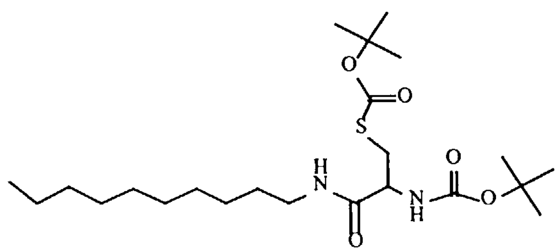

To a solution of N,S-bis-*tert*-butoxycarbonylcysteine (1 g; 3.11 mmol) in 6 ml dichloromethane and 200 µl tetrahydrofurane 430 mg N-hydroxysuccinimide (3.73 mmol) were added. The reaction mixture was cooled to 0°C and 713 mg N,N'-dicyclohexylcarbodiimide (3.42 mmol) were added. After 2 h of reaction at 0°C, 680 µl decylamine (3.42 mmol) were introduced and the solution was agitated for 6 h at ambiant temperature. Then the reaction medium was filtered, evaporated to dryness and then taken up in 200 ml ethylacetate. The organic phase was washed in subsequent steps with 100 ml 5 % (w/w) sodium hydrogen carbonate, 100 ml water, 100 ml citric acid 0.5 M, 100 ml water, then dried over magnesium sulfate and then evaporated to dryness. The obtained residue was purified by silica chromatography (gradient 1 to 3 % methanol in dichloromethane) to obtain 1.27 g of a translucid oil (2.75 mmol, yield=88 %).

$C_{23}H_{44}N_2O_5S$; 460,68 g/mol

TLC: Rf (5% MeOH / 1% AcOH / $CH_2Cl_2$) = 0,6.

NMR$^1$H ($CDCl_3$); δ ppm: 0,89 (t, J=7 Hz, 3H, **CH3**); 1,21-1,38 (m, 14H, $CH_3$-(**CH$_2$**)$_7$-$CH_2CH_2NH$); 1,45 (s, 9H, NH-C(**CH$_3$**)$_3$); 1,51 (s, 9H, S-C(**CH$_3$**)$_3$); 1,42-1,58 (m, 2H, **CH$_2$**-$CH_2NH$), 3,08-3,32 (m, 4H, **CH$_2$**-S, **CH$_2$**-NHCO); 4,27 (dd, $J_1$=13 Hz, $J_2$=8 Hz, 1H, **CH**-CONH); 5,35-5,43 (brs, 1H, **NH**Boc); 6,28-6,37 (brs, 1H, **NH**COCH).

MS (FAB+), m/z : 461,3 [MH$^+$]; 405,3 [MH$^+$ - *t*-Butyl]; 361,3 [MH$^+$ - Boc]; 349,2 [MH$^+$ - 2 *t*-Butyl]; 305,2 [MH$^+$ - *t*-Butyl - Boc]; 261,2 [MH$^+$ - 2Boc].

Step 4

Manufacture of S-*tert*-butoxycarbonylcysteine-decylamide

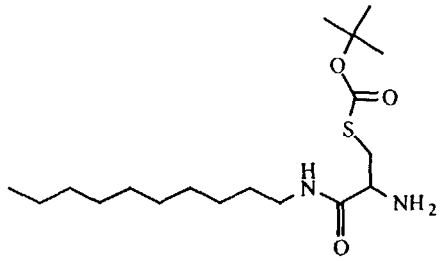

461 mg N,S-bis-tert-butoxycarbonyl-L-cysteine-decylamide (1 mmol) were dissolved in 3 ml 2N HCl/AcOEt at 0°C and agitated for 1 h. The solution was evaporated to dryness and then taken up in 100 ml ethylacetate. The organic phase was washed twice with 5 % (w/w) sodium hydrogen carbonate, dried over magnesium sulfate and then evaporated to dryness. The obtained oil was purified by silica chromatography (gradient 0 to 3 % methanol in dichloromethane) to obtain 233 mg of a translucid oil (0.65 mmol, yield=65 %).

$C_{18}H_{36}N_2O_3S$; 360,56 g/mol

TLC: Rf (10% MeOH / $CH_2Cl_2$) = 0,7.

NMR$^1$ H ($CDCl_3$); δ ppm: 0,89 (t, J=6 Hz, 3H, C**H3**); 1,10-1,40 (m, 14H, $CH_3$-(C**H$_2$**)$_7$-$CH_2CH_2$NH); 1,41-1,58 (m, 2H, C**H$_2$**-$CH_2$NH); 1,50 (s, 9H, S-C(C**H$_3$**)$_3$); 1,62 (brs, 2H, N**H$_2$**); 3,06 (dd, $J_1$=14 Hz, $J_2$=8 Hz, 1H, C**H**-S); 3,24 (dt, $J_1$=7 Hz, $J_2$=7 Hz, 2H, C**H$_2$**-NHCO); 3,37 (dd, $J_1$=14 Hz, $J_2$=4 Hz, 1H, C**H**-S); 3,55 (dd, $J_1$=8 Hz, $J_2$=4 Hz, 1H, C**H**-CONH); 7,3-7,4 (brs, 1H, N**H**).

MS (FAB+), m/z: 361,2 [$MH^+$]; 305,1 [$MH^+$ - *tert*-Butyl]; 261,1 [$MH^+$ - Boc].

Step 5

Manufacture of N,N'-bis-*tert*-butoxycarbonyl-guanidinyl-S-*tert*-butoxycarbonylcysteine-decylamide

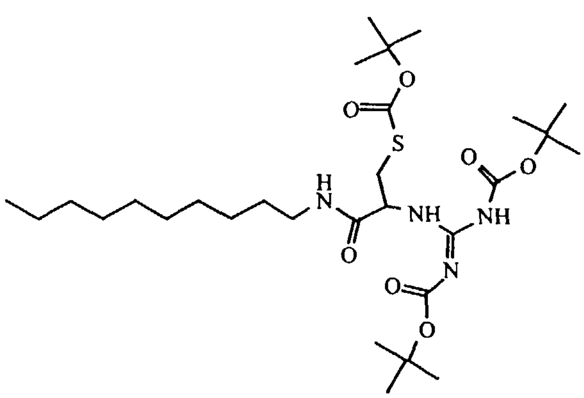

N,N'-bis-*tert*-butoxycarbonyl-pyrazole-1-carboxamidine (178 mg; 0.572 mmol) was added to a solution of S-*tert*-butoxycarbonylcysteine-decylamide (227 mg; 0.630 mmol) in 3 ml acetonitrile. The reaction mixture was stirred for 2 days at room temperarure and taken to dryness. The residue was purified by silicagel chromatography (10-15% ethyl acetate in hexane), leading to a white solid (281 mg; 0.47 mmol, 81%).

$C_{29}H_{54}N_4O_7S$; 602,84 g/mol

TLC: Rf (20% AcOEt / Hexane) = 0,6.
NMR$^1$H ($CDCl_3$); δ ppm: 0,9 (t, J=6 Hz, 3H, $\mathbf{CH_3}$); 1,18-1,40 (m, 14H, $CH_3$-$(\mathbf{CH_2})_7$-$CH_2CH_2NH$); 1,45-1,65 (m, 29H, 3 X -C$(\mathbf{CH_3})_3$, $\mathbf{CH_2}$-$CH_2NH$); 3,17-3,35 (m, 4H, $\mathbf{CH_2}$-S, $\mathbf{CH_2}$-NHCO); 4,8 (dd, $J_1$=11 Hz, $J_2$=6 Hz, 1H, **CH**-CONH); 6,98-7,07 (m, 1H, $CH_2CH_2$**NH**); 8,81 (d, J=7 Hz, **NH**-Boc); 11,36 (sl, 1H, **NH**-CN(NH)).
MS (FAB+), m/z : 603.3 [MH$^+$]; 547.2 [MH$^+$ - *t*-Butyl]; 503.3 [MH$^+$ - Boc]; 447.2 [MH$^+$ - *t*-Butyl - Boc]; 403.2 [MH$^+$ - 2Boc); 347.1 [MH$^+$ - *t*-Butyl - 2Boc]; 303.2 [MH$^+$ - 3Boc].

Step 6

Manufacture of Guanidyl-cysteine-decylamide ($C_{10}$-$C^{G+}$)

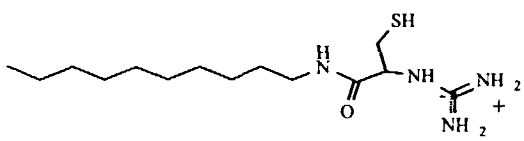

N,N'-bis-*tert*-butoxycarbonyl-guanidinyl-S-*tert*-butoxycarbonylcysteine-decylamide (60 mg; 0.1 mmol) was dissolved into 1 ml trifluoroacetic acid and stirred under argon atmosphere for 2 h at room temperature. The mixture was taken to dryness and the resulting colorless oil was dissolved into 1 ml perdeuteroethanol. The resulting 0.1 M stock solution was kept under argon atmosphere at -80°C. The thiol content was determined with Elmann's reagent following the method of Riddles et al. (87%).

$C_{14}H_{30}N_4OS$; 302,48 g/mol

NMR$^1$H ($CD_3CD_2OD$); δ ppm: 0,90 (t, J=6 Hz, 3H, $\mathbf{CH_3}$); 1,27-1,42 (m, 14H, $CH_3$-$(\mathbf{CH_2})_7$-$CH_2CH_2NH$); 1,50-1,61 (m, 2H, $\mathbf{CH_2}$-$CH_2NH$); 2,90-3,35 (m, 4H, $\mathbf{CH_2}$-S, $\mathbf{CH_2}$-NHCO); 4,32-4,40 (m, 1H, **CH**-CONH).

b) Oxydation rate of cysteine detergent $C_{10}$ -**C**$^{G+}$ into cystine lipid

($C_{10}$ -**C**$^{G+}$)$_2$ occurs faster in the presence of template DNA. The stock buffer solution (TrisHCl 15 mM pH 8.4) was saturated with oxygen by three vacuum/oxygen atmosphere cycles. $C_{10}$ -**C**$^{G+}$ was injected from a concentrated stock solution to a final concentration of 90 μM. For each time point, a 0.5 ml aliquot was removed and mixed with 0.5 ml 2XEllman's reagent. The remaining free thiol was quantitated spectrophotometrically according to Riddles et al. (1979 ). For the template experiment, pCMVluc plasmid DNA was added to a final concentration of 60 μM before addition of the detergent. The results of the experiment are shown in Fig. 3.

c) Transmission electron microscopy (Fig. 4)

Transmission electron microscopy of an unfiltered solution of ($C_{10}$ -**C**$^{G+}$)$_2$ cationic lipid / DNA particles showing (a) a very homogeneous population (b) of spheres (c, d) with a diameter of ca. 23 nm; scale bar corresponds to 100 nm for all pictures. Sample solution was prepared by adding the detergent (90 μM final concentration) to a 60 μM pCMVluc DNA solution in HEPES buffer (15 mM, pH 7.4) and leaving the solution in aerobic condition overnight. Carbon films

were prepared by sublimation on freshly cleaved mica and recovered by flotation on Cu/Rh grids (300 mesh, Touzard&Matignon, Courtaboeuf, France). After overnight drying, grids were kept on a blotting paper in a Petri dish. Immediately before sample addition, grids were glow-discharged (110 mV, 25 s). A drop (5 μl) of sample solution was left on the grid for 1 min. Complexes were negatively stained with 30 μl aqueous uranylacetate (1% w/w) for 20 s, and excess liquid was removed with blotting paper. Observations were performed at 80 kV with a Philips EM 410 transmission electron microscope. Similar results were obtained with complexes observed in 0.15 M NaCl. (Fig. 4).

d) Physical and chemical stability of the $(C_{10}$ -$\mathbf{C}^{G+})_2$ / DNA complexes (Fig. 5).

1% agarose gel electrophoresis (90 min at 8 V/cm in 40 mM Tris-acetate buffer pH 8) of 20 μl samples containing 0.4 μg (lanes 1-4) or 0.2 μg (lanes 5-8) pCMV-luc plasmid DNA. Complexes were made by addition of a 100-fold concentrated $C_{10}$ -$\mathbf{C}^{G+}$ detergent solution (90 μM final concentration) to a solution of plasmid (60 μM) in HEPES buffer (15 mM, pH 7.4). The solution was gently mixed once and left to oxydize for 24 h. Lane 1: 60 μM plasmid DNA. Lane 2: 60 μM oxydized $(C_{10}$ -$\mathbf{C}^{G+})_2$/DNA complexes. Lane 3: as lane 2 in 3 mM final sodium dodecylsulfate before loading. Lane 4: as lane 2 in 6 mM dithiothreitol before loading. Lane 5: 60 μM plasmid incubated for 1 h at 37 °C with an equivalent volume of DMEM cell culture medium containing 10% serum. Lane 6: 60p μ M oxydized $(C_{10}$ -$\mathbf{C}^{G+})_2$/DNA complexes incubated as lane 5. Lane 7: as lane 6 in 3 mM final sodium dodecylsulfate before loading. Lane 8:30 μM pCMV-luc plasmid DNA.

e) Nanosizing DNA particles condensed with $C_{10}$-$C^{G+}$

Complexes were formed with plasmid CMV-Luc (5.6 kbp), with λ phage DNA (48 Kbp) and with T4 DNA (167 Kbp) in a concentration of 15 μM bp in Tris-HCl (15 mM pH 8.4). After 24 h of incubation the measurements were carried out. The results are shown in Fig. 6 (Fig. 6A: phage lambda. Fig. 6B: phage T4. Fig. 6C: CMV-Luc. 1eq. = 30μM; 1.2 eq. = 36μM; 1.4 eq. = 42μM).

In case of monomolecular condensation of the DNA molecules the volume of the formed particles is directly proportional to the length of DNA: volume=constant x length of DNA (bp) .

In case of spheric particles the volume is proportional to the third power of the diameter (vol=4Pi$R^3$/3), which can be presented by the following equation: particle diameter = constant x (length of DNA (bp))$^{1/3}$ . When the minimum particle size derived from different DNAs (Fig. 7B) was plotted as a function of the cubic root of their size (Fig. 7A), one could draw a straight line through the points including the origin. This is the proof for monomolecular condensation.

f) Transfection of lipophilic alcohol cysteine amide ($C_{10}$-$C^{G+}$)/DNA complexes into the cell line BNLCL2

24 h before transfection the cells were distributed in a 24 well culture plate (50.000 cells per well. Cells were cultured in 75-$cm^2$ flask (Costar) in Dulbecco's modified Eagle's medium (DMEM, GIBCO), streptomycin at 100μg/ml (GIBCO), penicillin at 100 international units/ml (GIBCO); and glutamine (glutamax) at 0.286 g/ml (Lancaster) at 37°C in humidified 5% $CO_2$-containing atmosphere.

Complexes with the plasmid CMV-Luc were formed by adding an aliquot of a concentrated $C_{10}$-$C^{G+}$ solution to a solution of the plasmid (30 μM bp) in oxygen-saturated Tris-HCl 15 mM pH 8.4. The solution was then incubated in oxygen atmosphere for 24 h. Immediately before transfection, the medium was replaced by fresh medium without serum. 100 μl of the transfection solution containing the complexes were added per well (2 μg DNA). 3 h after the transfection 1 % serum was added, 1 h later 9 % serum. 6 h after transfection the transfection medium was replaced by a medium complemented to 10 % serum. 24 h later luciferase expression was determined with a commercially available kit (Promega) in a luminometer (Berthold). Each experiment was conducted in duplicate, the results are given as light units per mg protein (bicinchoninic acid *(BCA) test, Pierce)*. As an internal standard a DNA complex containing 10 charge equivalent of PEI (polyethylenimine, 25 kDa) was used (Fig. 8).

Example 2

Synthesis of a lipophilic alcohol cysteine ester detergent

The synthesis was carried out according to the scheme depicted in Fig. 9.

Step 1

Manufacture of N,S-Bis-tert-butoxycarbonyl-L-cysteine

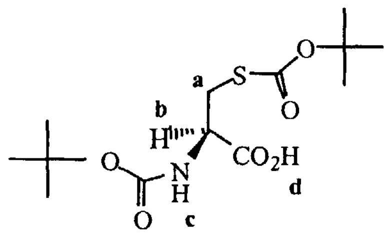

945 mg L-cysteine chlorohydrate (6 mmol) were dissolved in 10 ml deionized water in which gases were removed by vacuum. 2.625 g BocOBoc (12 mmol) in 10 ml THF and 5.4 ml triethylamine (39 mmol) were added to this solution. After two days of stirring under argon atmosphere and ambient temperature, the reaction medium was evaporated to a volume slightly below 10 ml. Then 10 ml deionized water were added and the aqueous phase was washed with ether in order to remove the remaining non-reacted BocOBoc. Next the aqueous phase was acidified to pH 3 with saturated citric acid and extracted with ether (3 x 60 ml). The ether phases were washed with 0.5 M citric acid (2 x 100 ml), dried over $MgSO_4$ and evaporated. Chromatography over a silica column (elution with a $CH_2Cl_2$/MeOH gradient containing 1 % acetic acid from 0 % to 2 %) yielded 1.34 g N,S-bis-tert-butoxycarbonyl-cysteine (4,2 mmol; yield=70 %).

TLC: Rf ($CH_2Cl_2$, MeOH 5%, AcOH 1%) = 0,5.

The structure of the obtained product was confirmed by nuclear magnetic resonance NMR$^1$H ($CDCl_3$) and the chemical shifts δ (ppm): 1,46 (s, 9H, N-Boc); 1,50 (s, 9H, S-boc); 3,22 (dd, $J_1$=15 Hz, $J_2$=7 Hz, 1H, Ha); 3,36 (dd, $J_1$=15 Hz, $J_2$=5 Hz, 1H, Ha); 4,34-4,57 (m, 1H, Hb); 5,45 (d, J=7,2 Hz, 1H, Hc); 6,34 (brs, 1H, Hd).

MS (FAB+) : 322,1 ($MH^+$).

Step 2

Manufacture of a lipophilic alcohol ester of N,S-Bis-tert-butoxycarbonyl-L-cysteine

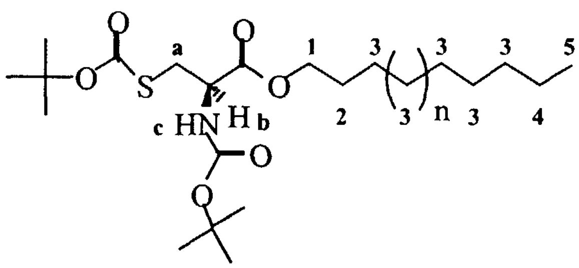

n = 0, 2, 3, 4

To a solution of lipophilic alcolhol (1.2 mmol) in 2.5 ml anhydrous dichloromethane 321 mg N,S-bis-tert-butoxycarbonyl-cysteine (1 mmol) and 24 mg 4-dimethylaminopyridine (0.2 mmol) were added. The solution was then cooled to 0°6 and 268 mg DCC (1.3 mmol; or EDC) were added. The solution was stirred for 2 h at 0°C and then over night at room temperature. Then the reaction medium was evaporated to dryness and the residue was resuspended in 10 ml ethylacetate. The suspension was filtered and the filtrate was diluted in 10 ml ethylacetate. The organic phase was washed with 20 ml distilled water, 20 ml aqueous 5 % sodium carbonate solution and 20 ml distilled water. The organic phase was dried over $MgSO_4$ and then evaporated to dryness. The obtained oil was finally chromatographed over a silica column (elution with hexane/ethylacetate from 0 % to 7 %) in order to obtain a translucid oil. In the case of C10 cysteine (n=2) a yield of 73 % was obtained. TLC (hexane, AcOEt 10%) Rf=0.50

The structure of the obtained product was confirmed by nuclear magnetic resonance NMR$^1$H ($CDCl_3$) the chemical shifts δ (ppm) were determined as follows: 0,90 (t, J=6,9 Hz, 3H, $H_5$); 1,25-1,42 (m, 12H, $H_3$); 1,46 (s, 9H, N-Boc); 1,51 (s, 9H, S-Boc); 1,56-1,71 (m, 4H, $H_2$, $H_4$); 3,18-3,38 (m, 2H, $H_a$); 4,14 (t, J=6,6 Hz, 2H, $H_1$); 4,50-4,58 (m, 1H, $H_b$); 5,30-5,38 (m, 1H, $H_b$); 5,30-5,38 (m, 1H, $H_c$).

MS (FAB+): 462,4 ($MH^+$).

Step 3

Manufacture of a lipophilic alcohol cysteine ester

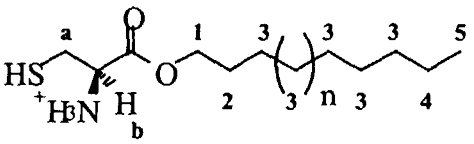 , $CF_3CO_2^-$ n = 0, 2, 3, 4

0.2 mmol of the lipophilic alcohol ester of N,S-bis-tert-butoxycarbonyl-L-cysteine dissolved in 1.5 ml trifluoroacetic acid were stirred in argon atmosphere for 1 h. The reaction medium was evaporated and the obtained translucid oil was dissolved in 1 ml deutero-ethanol and kept under argon atmosphere at -80°C.

The measurement of thiol groups with Ellman's reagent (Riddles et al., 1979) showed that the deprotection reaction was quantitative.

In the case of C10 cysteine (n=2):
The structure of the obtained product was confirmed by nuclear magnetic resonance NMR$^1$H ([$D_6$]ethanol) and the chemical shifts δ (ppm) were determined as follows: 0,91 (t, J=6,8 Hz, 3H, $H_5$); 1,24-1,48 (m, 14H, $H_3$, $H_4$); 1,66-1,78 (m, 2H, $H_2$); 3,06-3,22 (m, 2H, $H_a$); 4,18-4,40 (m, 3H, $H_b$, $H_1$).

MS (FAB+): 262,2 ($MH^+$).

b) Determination of the critical micelle concentration (c.m.c.) of the lipophilic alcohol cysteine esters with and without DNA

i) c.m.c. of a detergent with C8, C10, C11 and C12 cysteine

Measurement of the c.m.c. was acomplished with a Langmuir balance in a MES buffer (20 mM pH 6) containing 10 mM DTT. When representing the surface tension as a logarithmic function of the detergent concentration a break at the c.m.c. was observed (Fig. 10).

Applying this method c.m.c.'s of the compounds having different chain length (C8, C10, C11, C12) were determined.

| Cn-Cysteine | CMC |
|---|---|
| C8 | > 2 mM |
| C10 | 320 μM |
| C11 | 80 μM |
| C12 | 15 μM |

ii) c.m.c. of C10 and C11 cysteine with and without DNA. In an analogous manner as above the c.m.c. value of C10 cysteine and C11 cysteine was determined, the buffer containing calf thymus DNA at a concentration of 30 μM bp (Fig. 11A, B).

| CnCysteine | CMC without DNA | CMC with DNA |
|---|---|---|
| C10 | 320 μM | 50 μM |
| C11 | 80 μM | 32 μM |

c) Oxidation of C10 cysteine with and without DNA

The speed of the oxidation of C10 cysteine was determined by measuring the thiol groups, using Ellman's reagent (Riddles et al., 1979), as a function of time. The measurement was carried out in a solution of 60 μM C10 cysteine in MES 20 mM pH 6 without DNA or in the presence of 30 μM bp CMV-Luc (Fig. 12).

Example 3

Synthesis of a lipophilic alcohol guanidyl-cysteine ester detergent

The synthesis was carried out according to the scheme depicted in Fig. 13.

Step 1

Manufacture of S-*tert*-butoxycarbonylcysteine-decylester

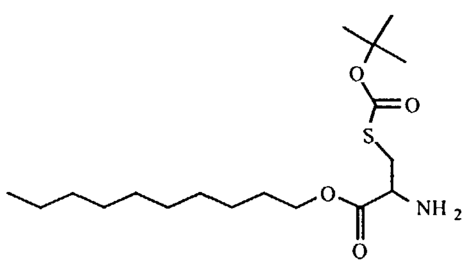

231 mg N,S-bis-*tert*-butoxycarbonyl-cysteine-decylester (0,5 mmol) were dissolved in 1.1 ml 2.2 N HCl/AcOEt. The solution was agitated for 1 h at ambient temperature and then evaporated to dryness. The obtained residue was taken up in 50 ml ethylacetate. The organic phase was washed twice with 25 ml 5 % (w/w) sodium hydrogencabonate, dried over magnesium sulfate and then evaporated to dryness. The obtained oil was purified by silica chromatography (gradient 0 to 3 % methanol in dichloromethane) to obtain 141 mg of a yellow oil (0.39 mmol, yield=78 %).

$C_{18}H_{35}NO_4S$; 361,55 g/mol

NMR$^1$H ($CDCl_3$); δ ppm: 0,90 (t, J=7 Hz, 3H, **CH3**); 1,20-1,42 (m, 14H, $CH_3$-O(**$CH_2$**)$_7$-$CH_2CH_2O$); 1,50 (s, 9H, C(**$CH_3$**)$_3$), 1,58-1,72 (m, 4H, **$CH_2$**-$CH_2O$, **$NH_2$**); 3,03 (dd, $J_1$=14 Hz, $J_2$=7 Hz, 1H, **CH**-S); 3,26 (dd, $J_1$=14 Hz, $J_2$=5 Hz, 1H, **CH**-S); 3,71 (dd, $J_1$=7 Hz, $J_2$=5 Hz, 1H, **CH**-COO); 4,13 (t, J=6 Hz, 2H, **$CH_2$**-O).

MS (FAB+), m/z: 362,2 [$MH^+$]; 306,2 [$MH^+$ - *t*-Butyl]; 262,2 [$MH^+$ - Boc].

Step 2

Manufacture of N,N'-bis-*tert*-butoxycarbonyl-guanidinyl-S-*tert*-butoxycarbonylcysteine-decylester

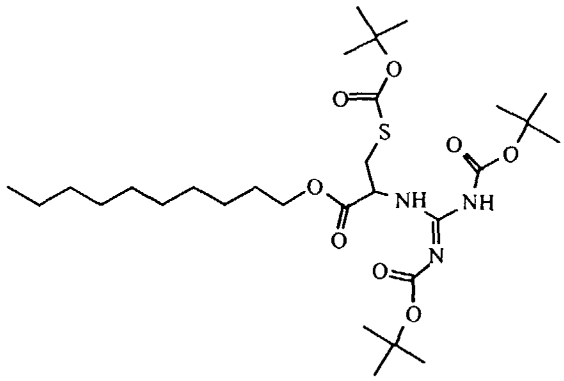

To a solution of 99 mg of S-*tert*-butoxycarbonylcysteine-decylester (0.275 mmol) in 1 ml acetonitril 78 mg N,N'-bis-tert-butoxycarbonyl-pyrazole-1-carboxamidine (0.25 mmol) were added. The reaction medium was agitated for 2 days at ambient temperature, then evaporated to dryness. The obtained residue was purified by silica chromatography (gradient 0 to 7 % ethylacetate in hexane) to obtain 127 mg of a translucid oil (0.21 mmol, yield=84 %).

$C_{29}H_{53}N_3O_8S$; 603,82 g/mol

TLC: Rf (10% AcOEt / Hexane) = 0,5.

NMR$^1$H ($CDCl_3$); δ ppm: 0,9 (t, J=7 Hz, 3H, **$CH_3$**); 1,25-1,42 (m, 14H, $CH_3$-(**$CH_2$**)$_7$-$CH_2CH_2O$); 1,479 (s, 9H, C(**$CH_3$**)$_3$); 1,493 (s, 9H, C(**$CH_3$**)$_3$); 1,497 (s, 9H, C(**$CH_3$**)$_3$); 1,58-1,73 (m, 2H, **$CH_2$**-$CH_2O$); 3,31 (dd, $J_1$=14 Hz, $J_2$=5 Hz, 1H, **CH**-S); 3,50 (dd, $J_1$=14 Hz, $J_2$=5 Hz, 1H, **CH**-S); 4,05-4,20 (m, 2H, **$CH_2$**-O); 5,15 (dt, $J_1$=7 Hz, $J_2$=5 Hz, 1H, **CH**-COO); 9,00 (d, J=5 Hz, 1H, **NH**-Boc); 11,42 (brs, 1H, **NH**-CN(NH)).

Step 3

Manufacture of guanidyl-cysteine-decylester

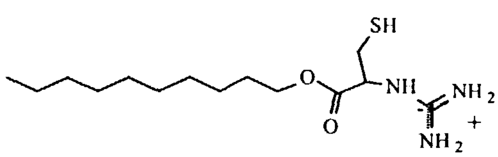

30 mg of N,N'-bis-*tert*-butoxycarbonyl-guanidinyl-S-*tert*-butoxycarbonylcysteine-decylester (0.05 mmol) dissolved in 1 ml trifluoroacetic acid were agitated under argon atmosphere for 2 h at ambient temperature. The reaction medium was then evaporated to dryness and the obtained translucid oil was dissolved in 1 ml deutero-ethanol. The solution was kept under argon atmosphere at -80°C. The yield of the reaction was determined by measuring the amount of thiol functions with Ellman's reagent (Riddles, et al., 1979). The reaction yield was 80 %.

$C_{14}H_{29}N_3O_2S$; 303,46 g/mol

NMR$^1$H ($CD_3CD_2OD$); δ ppm: 0,90 (t, J=6 Hz, 3H, $\mathbf{CH_3}$); 1,25-1,47 (m, 14H, $CH_3$-$(\mathbf{CH_2})_7$-$CH_2CH_2O$); 1,60-1,77 (m, 2H, $\mathbf{CH_2}$-$CH_2O$); 2,98-3,27 (m, 2H, $\mathbf{CH_2}$-S); 4,12-4,30 (m, 2H, $OCH_2$); 4,32-4,40 (m, 1H, **CH**-CONH).

Example 4

Synthesis of a lipophilic ornithyl-cysteine amide detergent

The synthesis was carried out according to the scheme depicted in Fig. 14.

Step 1

Manufacture of S-*tert*-butoxycarbonylcysteine hydrochloride

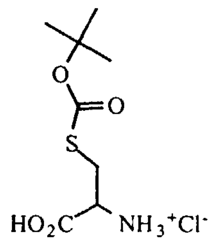

642 mg of N,S-bis-*tert*-butoxycarbonylcysteine (2 mmol) were dissolved in 3 ml 2.9 N HCl/AcOEt and the solution was agitated at ambient temperature for 20 min. 3 ml ether were added to the reaction medium and the obtained crystals were collected by filtration, washed with ether and finally dried under vacuum. Thereby 405 mg of white crystals were obtained (1.57 mmol, yield=79 %).

$C_8H_{16}NO_4ClS$; 257,74 g/mol

NMR$^1$H (DCl 0,1 M); δ ppm: 1,44 (s, 9H, $C(\mathbf{CH_3})_3$); 3,31 (dd, $J_1$=15 Hz, $J_2$=7 Hz, 1H, **CH**-S); 3,49 (dd, $J_1$=15 Hz, $J_2$=5 Hz, 1H, **CH**-S); 4,33 (dd, $J_1$=7 Hz, $J_2$=5 Hz, 1H, **CH**-$CO_2$).

MS (FAB+), m/z : 222,0 [$MH^+$]; 166,0 [$MH^+$ - *tert*-Butyl].

Step 2

Manufacture of N,N'-bis-*tert*-butoxycarbonylornithyl-S-*tert*-butoxycarbonylcysteine

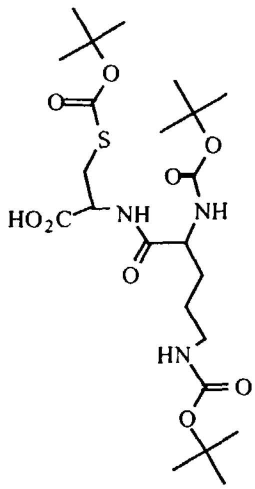

To a solution of N,N'-bis-*tert*-butoxycarbonylornithine (160 mg; 0.48 mmol) in 1 ml dichloromethane 60 mg of N-hydroxy-succinimide (0.52 mmol) were added. The reaction mixture was cooled to 0°C and 99 mg N,N'-dicyclohexylcarbodiimide (0.48 mmol) were added. After 2 h of agitating at 0°C, 130 mg hydrochloride S-*tert*-butoxycarbonylcysteine (0.50 mmol), which had been dissolved in 1 ml methanol containing 234 µl triethylamine (1.68 mmol), were added, then the solution was agitated over night at ambient temperature. The reaction medium was then evaporated to dryness, taken up in 10 ml ethylacetate and then filtered. After dilution with 50 ml ethylacetate, the filtrate was washed twice with 25 ml 5 % (w/w) citric acid, dried over magnesium sulfate and then evaporated to dryness. The obtained residue was purified by silica chromatography (gradient 2 to 15 % methanol in dichloromethane) to obtain 242 mg of a white solid (0.45 mmol, yield=94 %).

$C_{23}H_{41}N_3O_9S$; 535,66 g/mol

NMR$^1$H ($CD_3OD$); δ ppm: 1,42 (s, 9H, C(**$CH_3$**)$_3$); 1,45 (s, 9H, C(**$CH_3$**)$_3$); 1,46 (s, 9H, C(**$CH_3$**)$_3$); 1,47-1,88 (m, 4H, **$CH_2CH_2$**-$CH_2$NHBoc); 3,04 (t, J=6 Hz, 2H, **$CH_2$**-NHBoc); 3,13 (dd, $J_1$=14 Hz, $J_2$=7 Hz, 1H, **CH**-S); 3,45 (dd, $J_1$=14 Hz, $J_2$=4 Hz, 1H, **CH**-S); 4,0-4,1 (m, 1H, $CH_2CH_2$-**CH**-CONH); 4,40 (dd, $J_1$=7 Hz, $J_2$=4 Hz, 1H, **CH**-$CO_2$).

Step 3

Manufacture of N,N'-bis-*tert*-butoxycarbonylornithyl-S-*tert*-butoxycarbonylcysteine-n-alkylamide

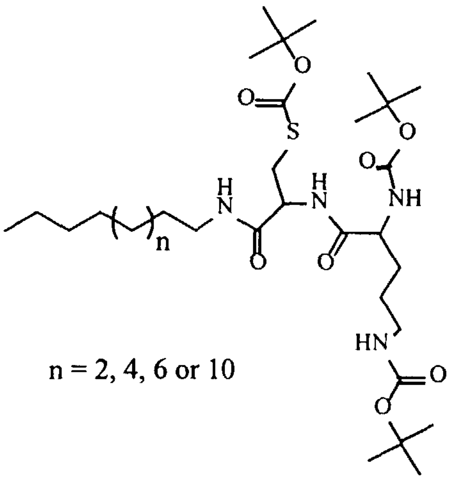

n = 2, 4, 6 or 10

To a solution of N,N'-bis-*tert*-butoxycarbonylornithyl-S-*tert*-butoxycarbonylcysteine (100 mg; 0.187 mmol) in 0.5 ml dichloromethane 26 mg N-hydroxysuccinimide (0.224 mmol) were added. The reaction mixture was cooled to 0°C and 42 mg N,N'-dicyclohexylcarbodiimide (0.205 mmol) were added. After 2 h of reaction at 0°C, n-alkylamine (0.205 mmol) was introduced and the solution was agitated over night at ambient temperature. Then the reaction medium was evaporated to dryness, taken up in 5 ml ethylacetate and then filtered. After dilution in 25 ml ethylacetate, the filtrate was washed in subsequent steps with 10 ml water, 10 ml 5 % (w/w) sodium hydrogencarbonate and 10 ml water, dried over magnesium sulfate and evaporated to dryness. The obtained residue was purified by silica chromatography (gradient 0 to 1 % methanol in dichloromethane) to obtain a white solid.

In the case of N,N'-bis-*tert*-butoxycarbonylornithyl-S-*tert*-butoxycarbonylcysteine-dodecylamide 85 mg of a white solid (0.121 mmol; yield=65 %) were obtained.

$C_{35}H_{66}N_4O_8S$; 703,00 g/mol

TLC: Rf (5% MeOH/$CH_2Cl_2$) = 0,5.

NMR$^1$H ($CD_3OD$); δ ppm: 0,89 (t, J=7 Hz, 3H, **$CH_3$**); 1,25-1,35 (m, 18H, $CH_3$-(**$CH_2$**)$_9$-$CH_2$ $CH_2$NH); 1,42 (s, 9H, C(**$CH_3$**)$_3$); 1,45 (s, 9H, C(**$CH_3$**)$_3$); 1,49 (s, 9H, C(**$CH_3$**)$_3$); 1,5-1,9 (m, 6H, **$CH_2CH_2$**-$CH_2$NHBoc, **$CH_2$**-$CH_2$NHCOCH); 3,00 (t, J=6 Hz, 2H, **$CH_2$**- NHBoc); 3,04-3,50 (m, 4H, **$CH_2$**-S, **$CH_2$**-NHCOCH); 3,94 (dd, $J_1$=8 Hz, $J_2$=5 Hz, 1H, $CH_2CH_2$-**CH**-CONH); 4,48 (dd, $J_1$=8 Hz, $J_2$=5 Hz, 1H, **CH**-CONH$CH_2$).

Step 4

Manufacture of ornithyl-cysteine-n-alkylamide

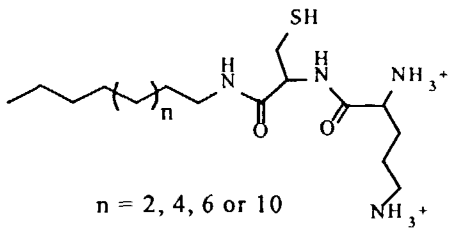

n = 2, 4, 6 or 10

0.11 mmol N,N'-bis-*tert*-butoxycarbonylornithyl-S-*tert*-butoxycarbonylcysteine-n-alkylamide dissolved in 1 ml trifluoroacetic acid were agitated under argon atmosphere at ambient temperature for 1.5 h. The reaction medium was evaporated and the obtained translucid oil was diluted in 1 ml deutero-ethanol. The solution was kept under argon atmosphere at -80°C. The yield of the reaction was determined by measuring the amount of thiol functions with Ellman's reagent (Riddles, et al., 1979). In the case of ornithyl-cysteine-dodecylamide, the yield was 84 %.

$C_{20}H_{42}N_4O_2S$; 402,64 g/mol

NMR$^1$H ($CD_3OD$); δ ppm: 0,89 (t, J=7 Hz, 3H, **$CH_3$**); 1,25-1,38 (m, 18H, $CH_3$-(**$CH_2$**)$_9$-$CH_2$ $CH_2$NH); 1,45-1,96 (m, 6H, **$CH_2CH_2$**-$CH_2$NHBoc, **$CH_2$**-$CH_2$NHCOCH); 2,80-3,50 (m, 6H, **$CH_2$**- $NH_3^+$, $CH_2$-S, **$CH_2$**-NHCO); 3,95-4,0 (m, 1H, $CH_2CH_2$-**CH**-CONH); 4,42-4,48 (m, 1H, **CH**-CONH$CH_2$).

Example 5

Synthesis of a lipophilic alcohol galactosyl-cysteine ester detergent

The synthesis was carried out according to the scheme depicted in Fig. 15.

Step 1

S-*tert*-butoxycarbonylcysteine-bromoacetamide-decylester

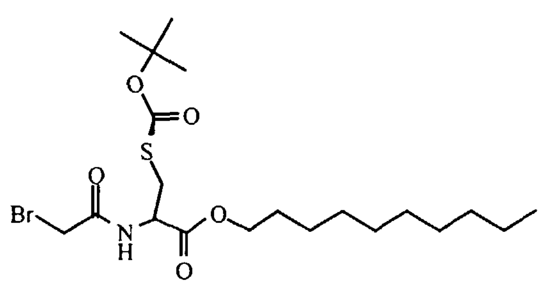

To a solution containing 108 mg of S-*tert*-butoxycarbonylcysteine-decylester (0.30 mmol) in 1 ml dichlormethane, 63 µl triethylamine (0.45 mmol) and then 86 mg bromoacetic acid anhydride (0.33 mmol) were added. The reaction medium was agitated for 1 h at ambient temperature and then evaporated to dryness. The obtained residue was purified by silica chromatography (gradient 5 to 10 % ethylacetate in hexane) to obtain 88 mg of a translucid oil (0.18 mmol, yield=61 %).

$C_{20}H_{36}BrNO_5S$; 482,48 g/mol

TLC: Rf (20% AcOEt / Hexane) = 0,5.

NMR[1]H ($CDCl_3$); δ (ppm) : 0,90 (t, J=7 Hz, 3H, **$CH_3$**); 1,25-1,40 (m, 14H, $CH_3$-(**$CH_2$**)$_7$-$CH_2CH_2O$); 1,50 (s, 9H, C(**$CH_3$**)$_3$), 1,60-1,75 (m, 2H, **$CH_2$**-$CH_2O$); 3,26 (dd, $J_1$=15 Hz, $J_2$=6 Hz, 1H, **CH**-S); 3,42 (dd, $J_1$=15 Hz, $J_2$=4 Hz, 1H, **CH**-S); 3,90 (s, 2H, **$CH_2$**-Br); 4,17 (t, J=7 Hz, **$CH_2$**-O); 4,79 (ddd, $J_1$=7 Hz, $J_2$=6 Hz, $J_3$=4 Hz, 1H, **CH**-COO); 7,20-7,35 (m, 1H, N**H**).

MS (FAB+), m/z : 482,1 and 484,1 [$MH^+$]; 426,1 and 428,1 [$MH^+$ - *t*-Butyl]; 382,1 and 384,1 [$MH^+$ - Boc].

Step 2

Manufacture of S-*tert*-butoxycarbonylcysteine-thiogalactosylacetamide-decylester

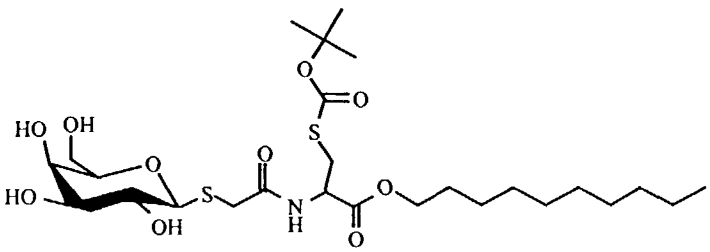

To a solution containing 48 mg S-*tert*-butoxycarbonylcysteine-bromoacetamide-decylester (0.1 mmol) in 1 ml dimethylformamide, 23 mg 1-thio-D-galactopyranose (0.1 mmol) were added. The solution was agitated for 3 h at ambient temperature and then evaporated to dryness. The obtained residue was purified by silica chromatography (gradient 3 to 20 % methanol in dichlormethane) to obtain 52 mg of a glassy solid (0.087 mmol, yield=87 %).

$C_{26}H_{47}NO_{10}S_2$; 597,79 g/mol

TLC : Rf (10% MeOH / $CH_2Cl_2$) = 0,4

NMR[1]H (CD3OD); δ ppm : 0,90 (t, J=7 Hz, 3H, **$CH_3$**); 1,25-1,43 (m, 14H, $CH_3$-(**$CH_2$**)$_7$-$CH_2CH_2O$); 1,48 (s, 9H, C(**$CH_3$**)$_3$), 1,58-1,73 (m, 2H, **$CH_2$**-$CH_2O$); 3,12 (dd, $J_1$=14 Hz, $J_2$=8 Hz, 1H, **CH**-S); 3,34 (s, 2H, CO-**$CH_2$**-S); 3,25-3,88 (m, 7H, **CH**-S, **$H_2$**, **$H_3$**, **$H_4$**, **$H_5$**, **$H_6$**); 4,08-4,17 (m, 2H, **$CH_2$**-O); 4,41 (d, J=9 Hz, **$H_1$**); 4,55-4,70 (m, 1H, **CH**-COO).

MS (FAB+), m/z : 620,2 [$MNa^+$]; 598,2 [$MH^+$]; 564,2 [$MNa^+$ - *t*-Butyl]; 542,2 [$MH^+$ -*t*-Butyl]; 520,2 [$MNa^+$ - Boc].

Step 3

Manufacture of cysteine-thiogalactosylacetamide-decylester

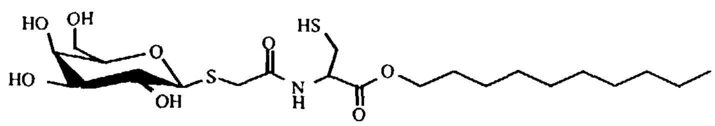

49 mg S-tert-butoxycarbonylcysteine-thiogalactosylacetamide-decylester (0.082 mmol) dissolved in 1 ml trifluoroacetic acid was agitated under argon atmosphere for 1.5 h at ambient temperature. The reaction medium was then evaporated to dryness and the obtained oil was dissolved in deuteroethanol. The solution was kept under argon atmosphere at -80°C.
The quantitative yield of the reaction was determined by measuring the amount of thiol functions with Ellman's reagent (Riddles, et al., 1979).

$C_{21}H_{39}NO_8S_2$; 497,67 g/mol

NMR$^1$H ($CD_3CD_2OD$); δ ppm : 0,90 (t, J=7 Hz, 3H, **CH3**); 1,20-1,43 (m, 14H, $CH_3$-($\mathbf{CH_2}$)$_7$-$CH_2CH_2O$); 1,60-1,76 (m, 2H, $\mathbf{CH_2}$-$CH_2O$); 2,85-3,08 (m, 2H, $\mathbf{CH_2}$-S); 3,35-4,00 (m, 8H, $\mathbf{H_2}$, $\mathbf{H_3}$, $\mathbf{H_4}$, $\mathbf{H_5}$, $\mathbf{H_6}$, CO-$\mathbf{CH_2}$-S); 4,07-4,28 (m, 2H, $\mathbf{CH_2}$-O); 4,43 (d, J=9 Hz, 1**H**); 4,58-4,70 (m, 1H, **CH**-COO).

MS (FAB+), m/z: 520 [$MNa^+$]; 498 [$MH^+$].

Example 6

In order to form monodisperse DNA transfection particles which are stabilised by non-lipid molecules, an alternative vector type, based on spermine-N1, N12-bis-cysteineamide ($SC_2$) was synthesized.

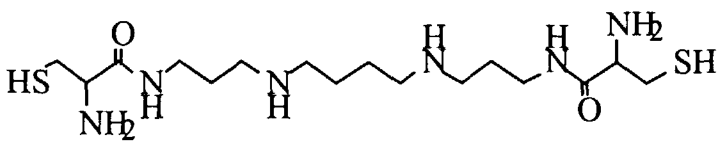

$SC_2$

This molecule is composed of a spermine molecule linked via an amide bond to 2 cysteine molecules having 2 free thiol functions.

a) Synthesis of spermine-N1, N12-bis-cysteineamide

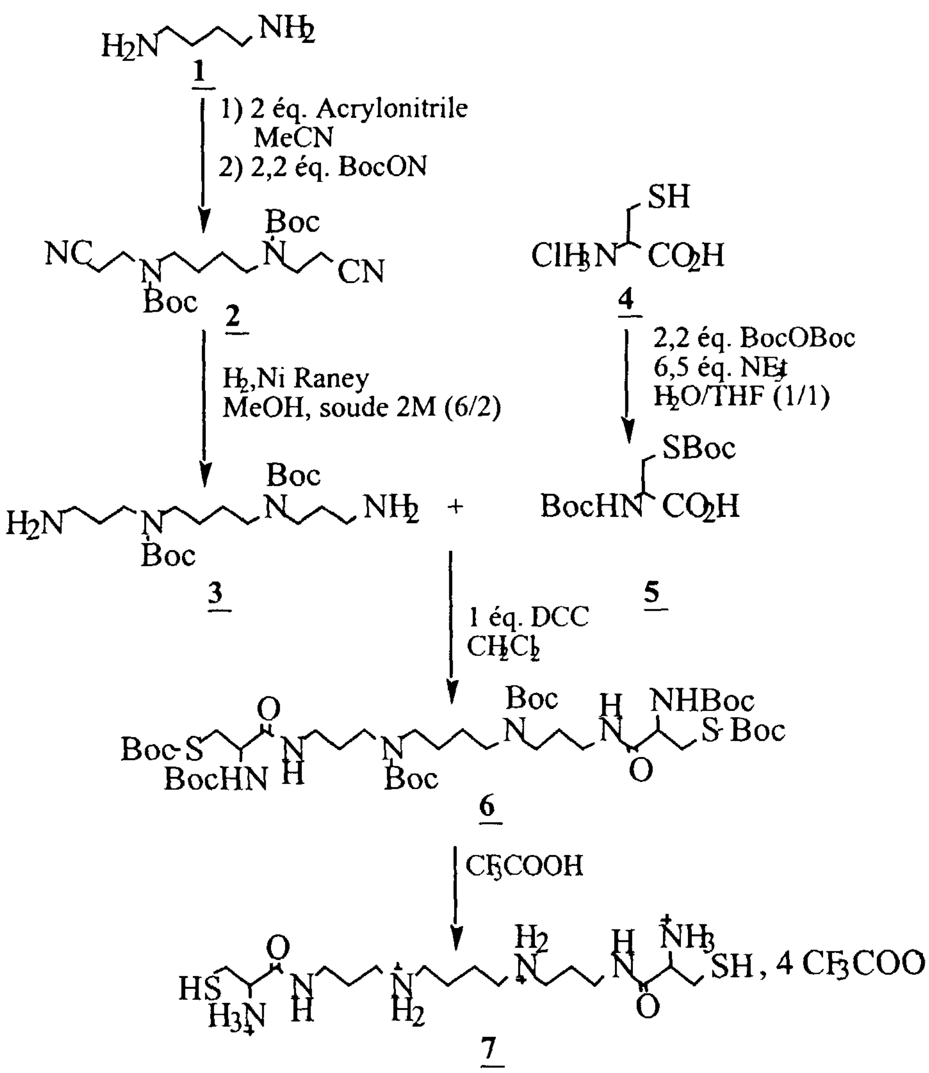


N,N'-bis-tert-butoxycarbonyl-N,N'-(2-cyanoethyl)-1,4-diaminobutane 2.

A solution containing 2 ml 1.4-diaminobutane 1 (20 mmol), 2.63 ml acrylonitrile (40 mmol) in 10 ml acetonitrile was stirred at ambient temperature under argon atmosphere and exposed to under darkness. After 18 h of stirring, 10.835 g BocON (44 mmol) and 6.2 ml triethylamine (44 mmol), were introduced into the solution. After three days the reaction medium was diluted in 100 ml ether. The organic phase was washed with 2 M NaOH 2 x 50 ml), 50 ml distilled water and 50 ml 5 % (w/w) citric acid. The organic phase was then dried over $MgSO_4$ and then evaporated to yield 4.27 g of a white solid. The latter was cristallized in ether which yielded 3.50 g of compound 2 (8.9 mmol, yield=44 %) in the form of white cristals.

$C_{20}H_{34}N_4O_4$; 394,52 g/mol.

TLC : Rf ($CH_2Cl_2$ 96%/MeOH 4%) = 0,4.

NMR $^1H$ ($CDCl_3$); δ ppm :1,29-1,65 (m, 22H, $(CH_2)_2$-**CH₂**-$CH_2$, C(**CH₃**)₃); 2,50-2,68 (m, 4H, **CH₂**-N(Boc)-$(CH_2)_2$-CN); 3,19-3,32 (m, 4H, N(Boc)-**CH₂**-$CH_2$-CN); 3,44 (t, J=6,7 Hz, 4H, **CH2**-CN). (The hydrogen atoms assigned to the signal are in bold print.)

N,N'-bis-tert-butoxycarbonyl-N,N'-(2-aminopropyl)-1,4-diaminobutane 3.

A solution containing 1.176 g N',N-Di-tert-butoxycarbonyl-N,N'-(2-cyanoethyl)-1,4-diaminobutane 2 (3 mmol) in 6 ml methanol and 2 ml 2 M NaOH (4 mmol) was stirred in the presence of a catalytic amount of Raney nickel under hydrogen atmosphere. After 4 h of stirring the nickel was removed by celite filtration. The filtrate was evaporated and the obtained residue resuspended in 50 ml dichloromethane. The organic phase was then washed with 50 ml 2 M *NaOH*, dried over $MgSO_4$ and then evaporated to obtain 810 mg of compound 3 (2 mmol; yield=67 %) in form of a yellow viscous liquid.

$C_{20}H_{42}N_4O_4$; 402,58 g/mol.

TLC: Rf ($CH_2Cl_2$ 20%/MeOH 60%/ $NEt_3$ 20%) = 0,4.
NMR$^1H$ ($CDCl_3$); δ ppm: 1,35-1,55 (m, 8H, $(CH_2)_2$-**CH₂**-$CH_2$, N**H₂**); 1,45 (s, 18H, 2xC$(CH_3)_3$; 1,64 (quint., J=6,8 Hz, 4H, **CH₂**-$CH_2$-$NH_2$); 2,69 (t, J=6,7 Hz, 4H, **CH₂**-$NH_2$); 3,10-3,32 (m, 8H, **CH₂**-N(Boc)-**CH₂**).

Spermine-N4,N9-bis-tert-butoxycarbonyl-N1,N12-bis-cysteine (N,S-bis-tert-butoxycarbonyl)amide 6.

To a solution containing 242 mg N,N'-bis-tert-butoxycarbonyl-N,N'-(2-aminopropyl)-1,4-diaminobutane 3 (0.6 mmol) and 388 mg N,S-Bis-tert-butoxycarbonyl-cysteine (1.2 mmol) in 1.5 ml dichloromethane, 248 mg DCC (1.2 mmol) dissolved in 1.5 ml dichloromethane were added. The solution was stirred for one day filtered in order to remove the formed DCU and then evaporated. The residue was resuspended in 100 ml ethylacetate, washed with distilled water (100 ml), an aqueous sodium carbonate solution 0.5 M (100 ml), an aqueous saturated NaCl solution (100 ml), an aqueous citric acid solution 0.2 M (100 ml) and finally with an aqueous saturated NaCl solution;100 ml). The organic phase was dried over $MgSO_4$ and then evaporated. The product was then run over a silica column in order to obtain 401 mg of compound 6 (0.4 mmol; yield=66 %) in the form of a white solid.

$C_{46}H_{84}N_6O_{14}S_2$; 1009,35 g/mol.

TLC : Rf ($CH_2Cl_2$ 97%/MeOH 3%) = 0,5.

NMR$^1$ H ($CDCl_3$); δ ppm: 1,50-1,89 (m, 8H, 2x$(CH_2)_2$-**CH₂**-$CH_2$, 2x**CH₂**-$CH_2$-NH-CO); 1,44 (s, 18H, 2xC$(CH_3)_3$); 1,45 (s, 18H, 2xC$(CH_3)_3$); 1,50 (s, 18H, 2xC$(CH_3)_3$); 3,04-3,32 (m, 16H, $(CH_2)_3$-**CH₂**-NBoc,**CH₂**-$(CH_2)_2$-NH-CO, **CH₂**-NH-CO, **CH₂**-S-CO); 4,23-4,40 (m, 2H, **CH**-$CH_2$-S-Boc); 5,37-5,54 (m, 2H, N**H**-Boc); 7,32-7,46 (m,2H, CH2-N**H**-CO).

MS (FAB+): 1009,3 ($MH^+$); 909,2 ($MH^+$-Boc); 809,3 ($MH^+$-2Boc); 709,2 ($MH^+$-3Boc); 609,2 ($MH^+$-4Boc): 509,2 ($MH^+$-5Boc); 409,1 ($MH^+$- 6Boc).

Spermine-N1,N12-bis-cysteineamide 7.

50 mg of compound 6 (0.05 mmol) dissolved in 1 ml trifluoroacetic acid and 1 ml dichloromethane were stirred under argon atmosphere for 2 h. The reaction medium was then evaporated and 85 mg of a yellowish viscous liquid is obtained. The product was kept in the presence of ca. 11 equivalents of trifluoroacetic acid in order to avoid oxydation of the functional thiol groups. The measurement of thiol groups with Ellman'sreagent (Riddles et al., 1979) showed that

the deprotection reaction was quantitative.

$C_{16}H_{36}N_6O_2S_2$; 408,64 g/mol.

NMR[1] H ($D_2O$); δ (ppm): 1,64-1,96 (m, 8H, $(CH_2)_2$-$\mathbf{CH_2}$-$CH_2$, $\mathbf{CH_2}$-$CH_2$-NH-CO); 2,90-3,09 (m, 12H, $CH_2)_3$-$\mathbf{CH_2}$-NBoc, $\mathbf{CH_2}$-$(CH_2)_2$-NH-CO, $\mathbf{CH_2}$-NH-CO); 3,21-3,37 (m, 4H, $\mathbf{CH_2}$-SH); 4,04-4,15 (m, 2H, C**H**-$CH_2$-SH).

MS (FAB+) : 410,1 ($MH^+$).

Biophysical characterisation of spermine-N1,N12-bis-cysteineamide

a) UV spectroscopy

The characterisation of the formed particles by UV spectroscopy was performed for different reasons. DNA in solution has a characteristic absorption spectrum between 240 and 310 nm. The absorption (optical density) has a maximum at 260 nm and 0 towards 310 nm. A change in DNA conformation should change the profile of the absorption spectrum. Moreover, the formation of particles results in an enhancement of the diffusion which can be perceived in UV by an enhancement of optical density. When the wave length is adjusted to a value slightly above 310 nm (which is the wave length at which the DNA in solution does not absorb) one can monitor the effect of particle formation.

Calf thymus DNA was complexed with spermine under conditions optimal for obtaining toroid structures as described by Naroditsky et al. (76 μM DNA bases, 65 mM spermine, 25 mM NaCl). The absorption observed between 230 and 330 nm increased during 10 min while complexation of DNA took place. Consequently, a reduction of the optical density could be detected in the range of 260 nm, which is the absorption maximum of DNA), while it remained stable towards 300 nm. Finally the absorption stabilised after one and a half hours (Fig.16A).

When the NaCl concentration was raised to 100 mM, the observed spectrum was identical with the spectrum of the DNA that had been observed before complexation with spermine (Fig. 16B).
It could be concluded from the above experiment that the condensation with spermine is visible in UV. Moreover it could be shown that a reversion of the process takes place when the ionic strength of the medium is raised.

The same experience as with the above experiments was made in the presence of spermine-N1,N12-bis-cysteineamide ($SC_2$) instead of spermine at the identical concentration (Fig. 16C).

The complexation kinetics of DNA by $SC_2$ is comparable to that obtained with spermine. The determination of thiol functions with Ellman's reagent (Riddles at al., 1979) showed that after 10 min 50% of the thiol groups were oxydised, when no oxygen was present except for the oxygen in the water (the solubility of oxygen in an 1 M NaCl aqueous solution is 0.8 mM). Furthermore, augmentation of the NaCl concentration one and a half hours after mixing $SC_2$ and DNA had only a minor effect on the absorption spectrum as compared with spermine. At 100 mM NaCl, the absorption at 330 nm was 81 % as compared to the maximum optical density observed at 25 mM NaCl; at 150 mM NaCl was still 71 %.

Consequently, $SC_2$ complexes and compacts DNA in the same manner as spermine. On the other hand, the formed particles seemed to be more stable vis a vis the ionic strength of the medium, which is in relation with the oxydation of the thiol functions.

b) Agarose gel electrophoresis

The stability of the particles formed either with spermine or with $SC_2$ (76 μM bases RSV-Luc, 65 μM spermine or $SC_2$, 25 mM NaCl, pH 6.5; 2 hrs oxydation) was tested by agarose gel electrophoresis (1 %; Tris buffer pH 6.5). It could be observed that the compacted DNA, which is more bulky and less charged, was migrating less or not at all in the agarose gel. (Fig. 17)

The particles formed with spermine were not stable under the conditions that were used for electrophoresis (identical migration of DNA alone and of DNA complexed with spermine; lanes 1 and 2). The particles formed with $SC_2$ were stable (lane 3). The DNA-$SC_2$ particles remained relatively stable even at elevated ionic strength (lane 5). The stability of the complexes is due to the oxydation of $SC_2$. Consequently, if one adds a reducing agent of the disulfide bridges to a solution of DNA complexed with $SC_2$, the complexes are no longer stable under the conditions used for electrophoresis.

References

1. Mulligan, R.C. The Basic Science of Gene Therapy. Science 260, 926-932 (1993).

2. Tang, M.X. & Szoka, F.C. The influence of polymer structure on the interactions of cationic polymers with DNA and morphology of the resulting complexes. Gene Therapy 4, 823-832 (1997).

3. Labatmoleur, F., Steffan, A.M., Brisson, C., Perron, H., Feugeas, O., Furstenberger, P., Oberling, F., Brambilla, E. & Behr, J.P. An electron microscopy study into the mechanism of gene transfer with lipopolyamines. Gene Therapy 3, 1010-1017 (1996).

4. Behr, J.P. DNA strongly binds to micelles and vesicles containing lipopolyamines or lipointercalants. Tet. Letters 27, 5861-5864 (1986).

5. Melnikov, S.M., Sergeyev, V.G. & Yoshikawa, K. Discrete coil-globule transition of large DNA induced by cationic surfactant. J Am Chem Soc 117, 2401-2408 (1995).

6. Behr, J.P. Synthetic gene-transfer vectors. Accounts Chem. Res. 26, 274-278 (1993).

7. Lehn, J.M. Supramolecular chemistry-Scope and perspectives: Molecules, supermolecules and molecular devices (Nobel lecture). Angew. Chem. Int. Ed. Engl. 27, 89-112 (1988).

8. Ringsdorf, H., Schlarb, B. & Venzmer, J. Molecular architecture and function of polymeric oriented systems: Models for the study of organization, surface recognition and dynamics of biomembranes. Angew. Chem. Int. Ed. Engl. 27, 113-158 (1988).

9. Gustafsson, J., Arvidson, G., Karlsson, G. & Almgren, M. Complexes between cationic liposomes and DNA visualized by cryo-TEM. Biochim. Biophys. Acta 1235, 305-312 (1995).

10. Mislick, K.A. & Baldeschwieler, J.D. Evidence for the role of proteoglycans in cation-mediated gene transfer. Proc Natl Acad Sci USA 93, 12349-12354 (1996).

11. Remy, J.S., Kichler, A., Mordvinov, V., Schuber, F. & Behr, J.P. Targeted gene transfer into hepatoma cells with lipopolyamine-condensed DNA particles presenting galactose ligands: A stage toward artificial viruses. Proc Natl Acad Sci USA 92, 1744-1748 (1995).

12. Fields, B.N. & Knipe, D.M. Virology (Raven Press, New York, 1990).

13. Boussif, O., Lezoualch, F., Zanta, M.A., Mergny, M.D., Scherman, D., Demeneix, B. & Behr, J.P. A versatile vector for gene and oligonucleotide transfer into cells in culture and in vivo: Polyethylenimine. Proc Natl Acad Sci USA 92, 7297-7301 (1995).

14. Riddles, P.W., Blakeley, R.L. & Zerner, B. Ellman's reagent: 5, 5'-dithiobis(2-nitrobenzoic acid); a reexamination. Anal. Biochem. 94, 75-81 (1979).

15. Plank, C., Oberhauser, B., Mechtler, K., Koch, C., and Wagner, E. (1994) J. Biol. Chem. 269, 12918-12924.

16. Mechtler, K. and Wagner, E. (1997), New J. Chemistry 21, 105-111.

17. Gottschalk, S., Sparrow, J.T., Hauer, J., Mims, M.P., Leland, F.E., Woo, S.L.C. and Smith, L.C. (1996) Gene Therapy 3, 448-457.

18. Wyman, T.B., Nicol, F., Zelphati, O., Scaria, P.V., Plank, C., and Szoka, F.C. (1997) Biochemistry 36, 3008-3017.

19. Legendre, J. Y., and Szoka, F. C. (1993) Proc. Natl. Acad. Sci. U.S.A. 90, 893,.

20. Legendre, J.Y., and Supersaxo, A. (1995) Biochem. Biophys. Res. Commun. 217, 179-85.

21. Wilson, P.D., Firestone, R.A., and Lenard, J (1987) J. Cell.Biol. 104, 1123-1229.

22. Hughes, J.A., Aronsohn, A.I., Avrutskaya, A.V., and Juliano, R.L. (1996) Pharm. Res. 13, 404-410.

23. Remington's Pharmaceutical Sciences (1980), Mack Publishing Co., Easton, PA, Osol (ed.)

## Claims

1. A particle for transfecting higher eucaryotic cells with nucleic acid molecules *in vitro* and *in vivo* comprising one or more nucleic acid molecules condensed by organic cationic molecules, said particles being obtained by complexing the nucleic acid molecules with identical or different organic cationic precursor molecules without crosslinking nucleic acid molecules, and covalently linking the precursor molecules to each other on the the nucleic acid template.

2. A transfection particle according to claim 1 characterized in that the cationic molecules are lipids obtained by dimerization or oligomerization of cationic detergent precursor molecules.

3. A transfection particle according to claim 2 characterized in that the cationic detergent precursor molecules comprise:

   a) at least one function for binding to one or more other detergent molecules,

   b) at least one lipophilic residue,

   c) a non-toxic recipient backbone,

   d) a cationic group for binding to nucleic acid molecules.

4. A transfection particle according to claim 3 characterized in that the function of the cationic precursor detergent molecules for binding to other detergent molecules is a dimerizable or polymerizable function selected from thiols, acid hydrazides, aldehydes, amines, and ethylene residues that are suitably substituted to provide enamines.

5. A transfection particle according to claim 4 characterized in that the lipophilic residue is selected from lipophilic amides, esters or ethers.

6. A transfection particle according to claim 3 characterized in that the function for binding to nucleic acid molecules is selected from an amine or derivative thereof.

7. A transfection particle according to claim 6 characterized in that the function for binding to nucleic acid molecules is guanidine.

8. A transfection particle according to claim 1 characterized in that the organic cationic precursor molecule is represented by general formula I

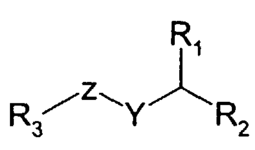

(I)

wherein

$R_1$ denotes ($C_1$-$C_{10}$-alkylene)-SH, wherein the alkylene radical may represent a straight chained or branched

hydrocarbon;

$R_2$ denotes $-NR_4R_5$, $-NHR_4R_5^+$, $-N(R_4)_2R_5^+$, $-C(=NR_4)NR_5R_6$, $-C(=X)-C_1-C_{10}$-alkylene, wherein the alkylene radical may represent a straight chained or branched hydrocarbon and may be substituted by up to four dialkyl amino groups or a thiomonosaccharide;

$R_3$ denotes $C_5-C_{30}$-alkyl, straight chained or branched and optionally substituted preferably with one or more halogen atom(s) or dialkyl amino group(s), or

$C_5-C_{30}$-alkenyl, straight chained or branched having up to ten C=C-double bonds and is optionally substituted preferably with one or more halogen atom(s) or dialkyl amino group(s), or

$C_5-C_{30}$-alkinyl, straight chained or branched having up to ten C=C-triple bonds and is optionally substituted preferably with one or more halogen atom(s) or dialkyl amino group(s), or

$C_6-C_{10}$-aryl optionally substituted, or

$C_7-C_{16}$-aralkyl optionally substituted, or a

$C_5-C_{30}$-alkyl-chain interrupted by up to 10 amino groups $-NR_4-$ and having optionally an amino-group which is optionally substituted by an amino acid;

$R_4$, $R_5$ and $R_6$ denote independently from each other hydrogen or $C_1-C_4$-alkyl;

X denotes O or S;

Y denotes C=O or C=S and

Z denotes O, S or $-NR_4-$.

**9.** A transfection particle according to claim 8 characterized in that the cationic precursor molecules correspond to general formula I wherein

$R_1$ denotes $(C_1-C_6$-alkylene)-SH, wherein the alkylene radical may represent a straight chained or branched hydrocarbon;

$R_2$ denotes $-NR_4R_5$, $-NHR_4R_5^+$, $-N(R_4)_2R_5^+$, $-C(=NR_4)NR_5R_6$, $-C(=X)-C_1-C_4$-alkylene, wherein the alkylene radical may represent a straight chained or branched hydrocarbon and may be substituted by up to four amino radicals $-NR_4R_5$ or a thiomonosaccharide;

$R_3$ denotes $C_5-C_{20}$-alkyl, straight chained or branched and optionally substituted preferably with F, Cl, Br or $-NR_4R_5$, or

$C_5-C_{20}$-alkenyl, straight chained or branched having up to five C=C-double bonds and is optionally substituted preferably with F, Cl, Br or $-NR_4R_5$, or

$C_5-C_{20}$-alkinyl, straight chained or branched having up to five C=C-triple bonds and is optionally substituted preferably with F, Cl, Br or $-NR_4R_5$, or

$C_6-C_{10}$-aryl optionally substituted preferably with $C_1-C_4$-alkyl ,F, Cl, Br or $-NR_4R_5$, or

$C_7-C_{14}$-aralkyl optionally substituted preferably with $C_1-C_4$-alkyl, F, Cl, Br or $-NR_4R_5$, or

a $C_5-C_{20}$-alkyl chain interrupted by up to 10 amino groups $-NR_4-$ and having optionally a -amino-group which is optionally substituted by an amino acid;

$R_4$, $R_5$ and $R_6$ denote independently from each other hydrogen or $C_1-C_4$-alkyl;

X denotes O or S;

Y denotes C=O or C=S and

Z denotes O, S or $-NR_4-$.

**10.** A transfection particle according to claim 8 characterized in that the cationic precursor molecules correspond to general formula I wherein

$R_1$ denotes ($C_1$-$C_4$-alkylene)-SH, wherein the alkylene radical may represent a straight chained or branched hydrocarbon;

$R_2$ denotes $-NR_4R_5$, $-NHR_4R_5^+$, $-N(R_4)_2R_5^+$, $-C(=NR_4)NR_5R_6$, $-C(=X)-C_1-C_4$-alkyl, wherein the alkyl radical may represent a straight chained or branched hydrocarbon and may be substituted by up to four amino radicals $-NR_4R_5$, or a thiomonosaccharide;

$R_3$ $C_5$-$C_{12}$-alkyl, straight chained or branched and optionally substituted preferably with F, Cl, Br or $-NH_2$, or a

$C_5$-$C_{15}$-alkyl chain interrupted by up to 7 amino groups $-NR_4-$ and having optionally a -amino-group which is optionally substituted by the amino acid cysteine;

$R_4$, $R_5$ and $R_6$ denote independently from each other hydrogen or methyl, ethyl, propyl, iso-propyl, n-butyl, iso-butyl or tert.-butyl;

X denotes O or S;

Y denotes C=O or C=S and

Z denotes O, S or $-NR_4-$.

**11.** A transfection particle according to claim 8 characterized in that the cationic precursor molecules correspond to the general formula I wherein

$R_1$ denotes $-CH_2-SH$;

$R_2$ denotes $-NH_2$, $-NH_3^+$, $-C(=N^+H_2)NH_2$, $-C(=O)-C_1-C_4$-alkyl straight chained or branched and optionally substituted with F, Cl, Br or $-NH_2$.
or an ornithine radical or a S-galactosyl radical;

$R_3$ denotes a $C_6$-$C_{15}$-alkyl radical straight chained or branched and optionally substituted preferably with F, Cl, Br or $-NH_2$;

Y denotes C=O;

Z denotes O or -NH-.

**12.** A transfection particle according to one of claim 8 to 11 characterized in that $R_2$ is guanidine ornithine.

**13.** A transfection particle according to one of claim 8 to 12 characterized in that $R_3$ is a decyl radical.

**14.** A transfection particle according to one of claim 8 to 11 characterized in that $R_1$ is a methylenethiol, $R_2$ is a guanidine, $R_3$ is a straight chained decyl radical, Y is a carbonyl, Z is an amine, and pharmaceutically acceptable salts thereof.

**15.** A transfection particle according to claim 14 characterized in that the cationic molecule is N-decyl-2-guanidinium-cysteine.

**16.** A transfection particle according to one of claim 8 to 11 characterized in that $R_1$ is a methylenethiol, $R_2$ is an orni-

thine, $R_3$ is a decane, Y is a carbonyl, Z is an amine, and pharmaceutically acceptable salts thereof.

**17.** A transfection particle according to claim 16 characterized in that the cationic molecule is N-decyl-2-ornithinyl-cysteine.

**18.** A transfection particle according to one of claim 8 to 11 characterized in that the monosaccharide which is bonded via a sulfur atom is selected from the group consisting of galactose, lactose, glucose, arabinose , fructose, sorbose, xylose, ribose, mannose each of them in their D- or L-form.

**19.** A transfection particle according to claim 1 characterized in that the cationic precursor molecule is a polyamine.

**20.** A transfection particle according to claim 19 characterized in that the cationic precursor molecule is a spermine derivative

**21.** A transfection particle according to claim 20 characterized in that the cationic precursor molecule is spermine-N1,N12-bis-cysteineamide.

**22.** A transfection particle according to claim 1 to 21 characterized in that the linkage between the cationic molecules is degradable under cellular conditions.

**23.** A transfection particle according to claim 1 which comprises a single nucleic acid molecule.

**24.** A transfection particle according to claim 1 or 23 wherein the nucleic acid molecule is a DNA molecule.

**25.** A transfection particle according to claim 24 wherein the DNA molecule is a plasmid.

**26.** A transfection particle according to claim 1 characterized in that the nucleic acid molecule is an RNA molecule.

**27.** A transfection particle according to any one of claims 1 to 26 characterized in that it carries one or more cellular targeting functions and/or one or more functions capable of facilitating endocytosis.

**28.** A transfection particle according to claim 27 characterized in that the targeting function is provided by a cellular protein ligand.

**29.** A transfection particle according to claim 27 characterized in that the targeting function is provided by a sugar residue.

**30.** A transfection particle according to claim 29 characterized in that the sugar is galactose.

**31.** A transfection particle according to claim 1 characterized in that carries one or more endosomolytic functions.

**32.** A transfection particle according to claim 1 characterized in that the endosomolytic function is provided by a fusogenic peptide.

**33.** A method for preparing transfection particles according to any of claims 1 to 32, characterized in that cationic precursor molecules are added to nucleic acid molecules in a suitable buffer, allowed to form complexes with the nucleic acid and allowed to covalently link to identical or different cationic precursor molecules on the nucleic acid template.

**34.** A method according to claim 33 characterized in that the cationic precursor molecules are lipophilic and are allowed to covalently link under mild oxidative conditions.

**35.** A pharmaceutical composition comprising a pharmaceutically effective amount of the transfection particle of claim 1 wherein the nucleic acid molecule is therapeutically active.

**36.** A pharmaceutical composition of claim 35 wherein the nucleic acid molecule is a plasmid encoding a therapeutically active protein.

**37.** A kit of parts comprising one or more nucleic acid molecules, one or more cationic precursor molecules, suitable buffers, and other reagents or mechanical devices that are useful for preparation, purification and *in vitro* or *in vivo* application of a transfection particle of any one of the claims 1 to 32.

**38.** A kit of parts according to claim 36 comprising in addition or more functions for cellular targeting.

**39.** A kit of parts according to claim 36 or 37 comprising in addition an endosomolytic function.

Fig. 1

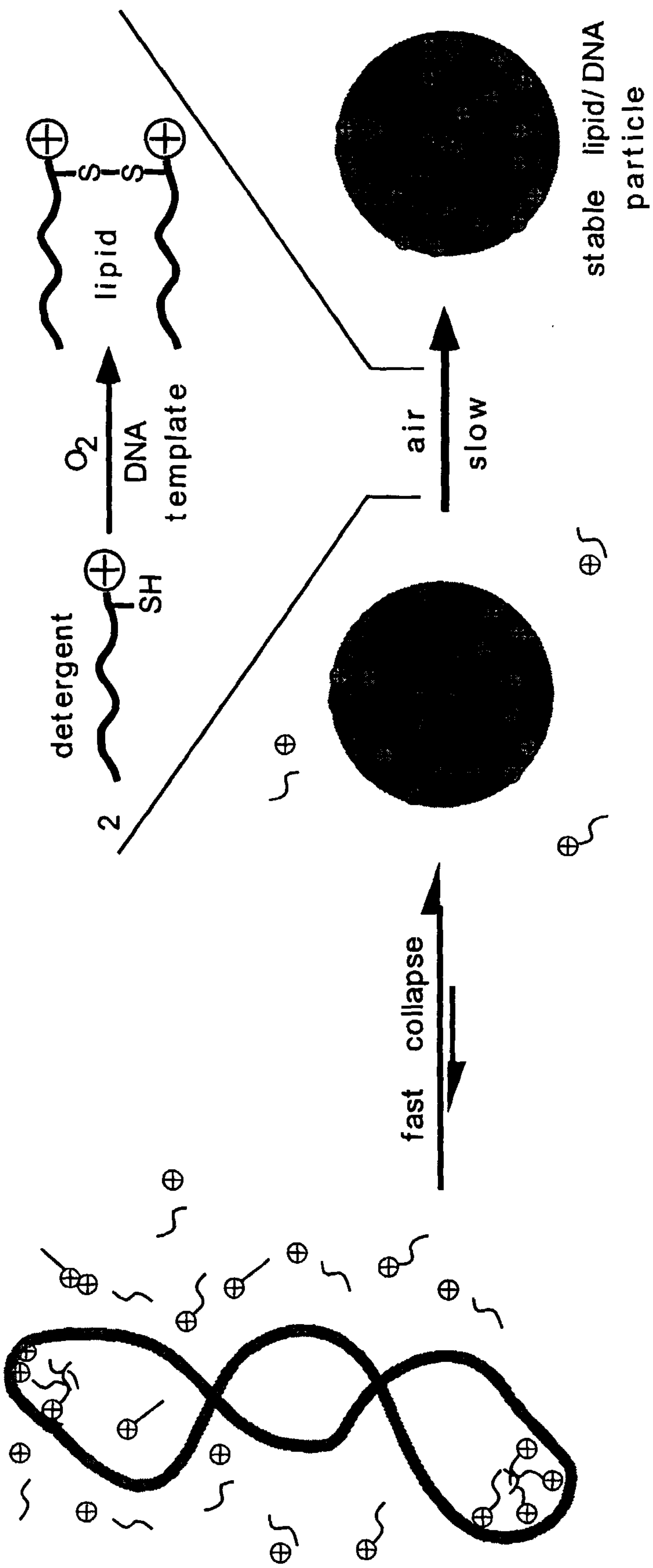
detergent
SH
2
O2
DNA
template
lipid
S
S
air
slow
fast
collapse
stable lipid/DNA
particle

Fig. 2

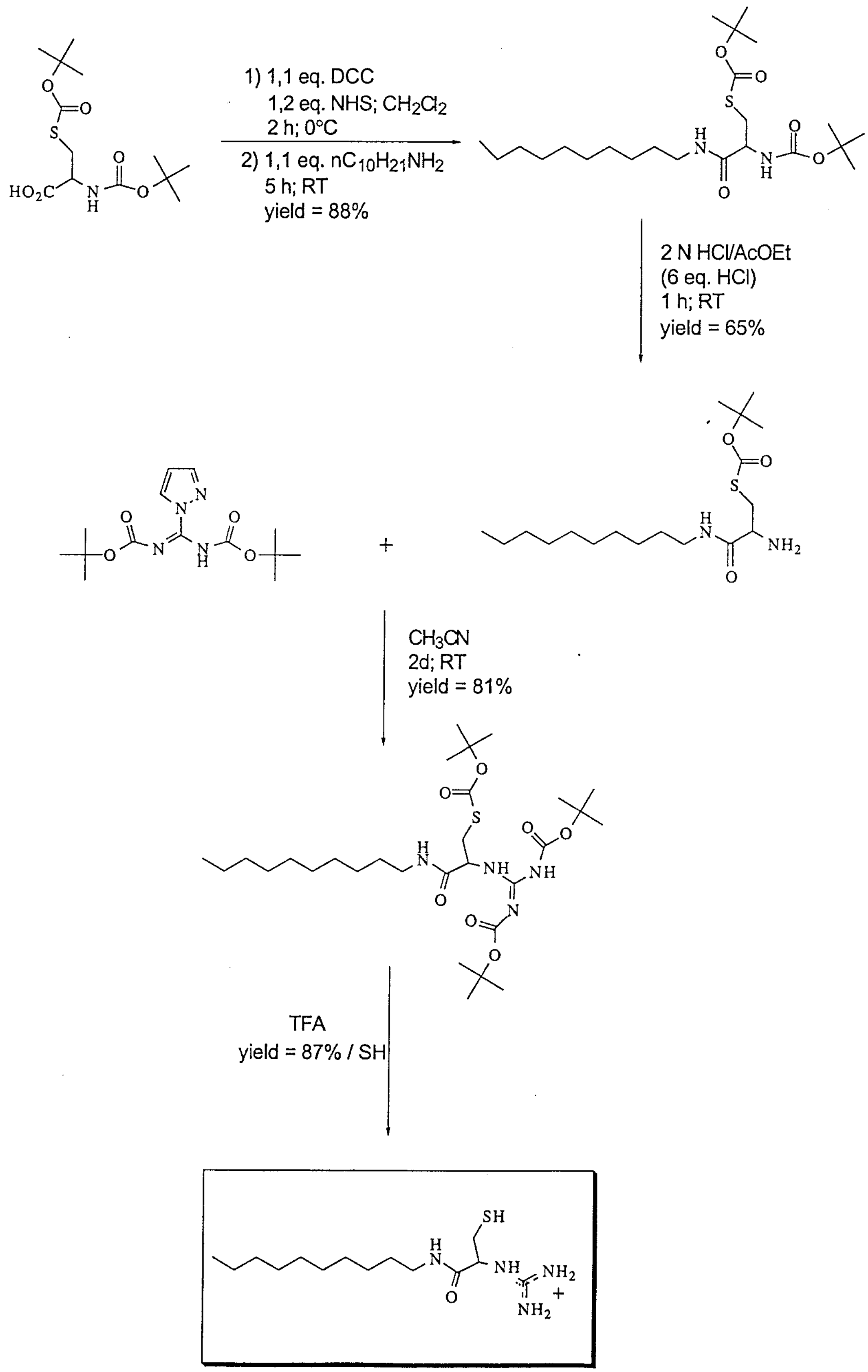
1) 1,1 eq. DCC
1,2 eq. NHS; $CH_2Cl_2$
2 h; 0°C
2) 1,1 eq. $nC_{10}H_{21}NH_2$
5 h; RT
yield = 88%
2 N HCl/AcOEt
(6 eq. HCl)
1 h; RT
yield = 65%
+
$CH_3CN$
2d; RT
yield = 81%
TFA
yield = 87% / SH

Fig. 3

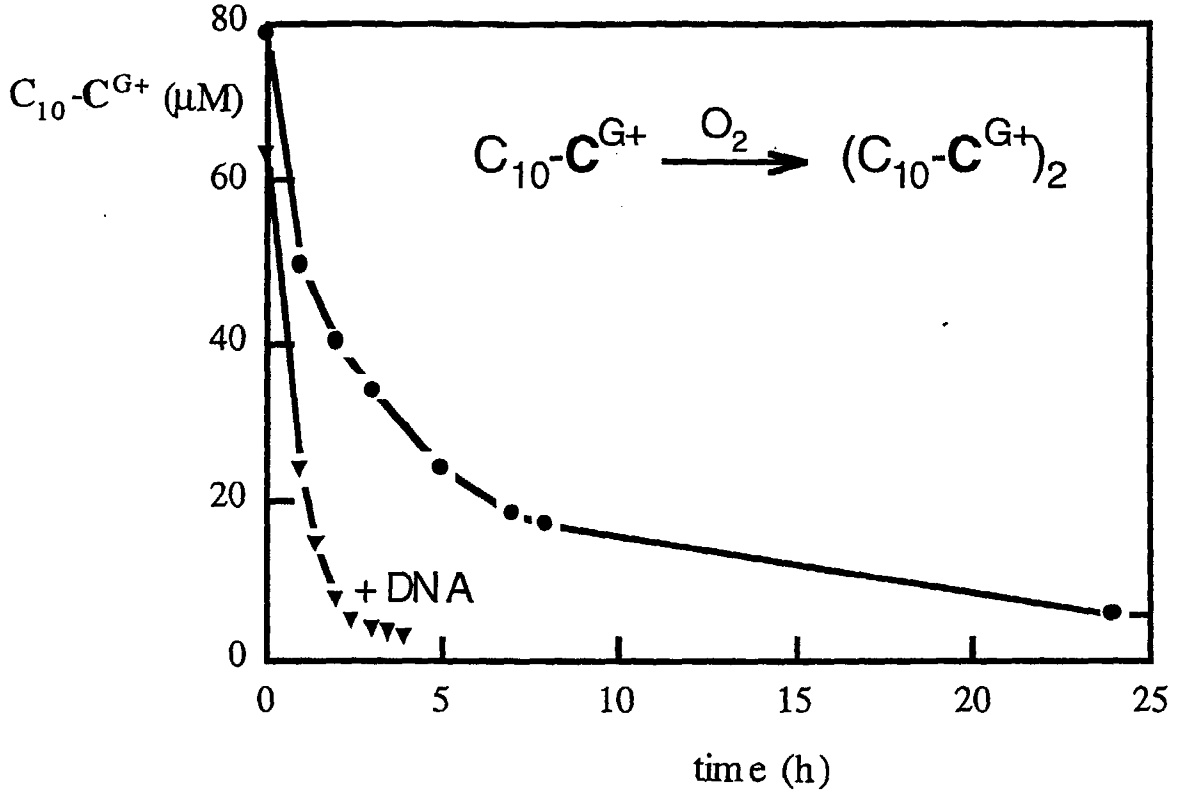
80
60
40
20
0
$C_{10}$-$C^{G+}$ (µM)
$C_{10}$-$C^{G+}$ $\xrightarrow{O_2}$ $(C_{10}$-$C^{G+})_2$
+ DNA
0
5
10
15
20
25
time (h)

Fig. 4

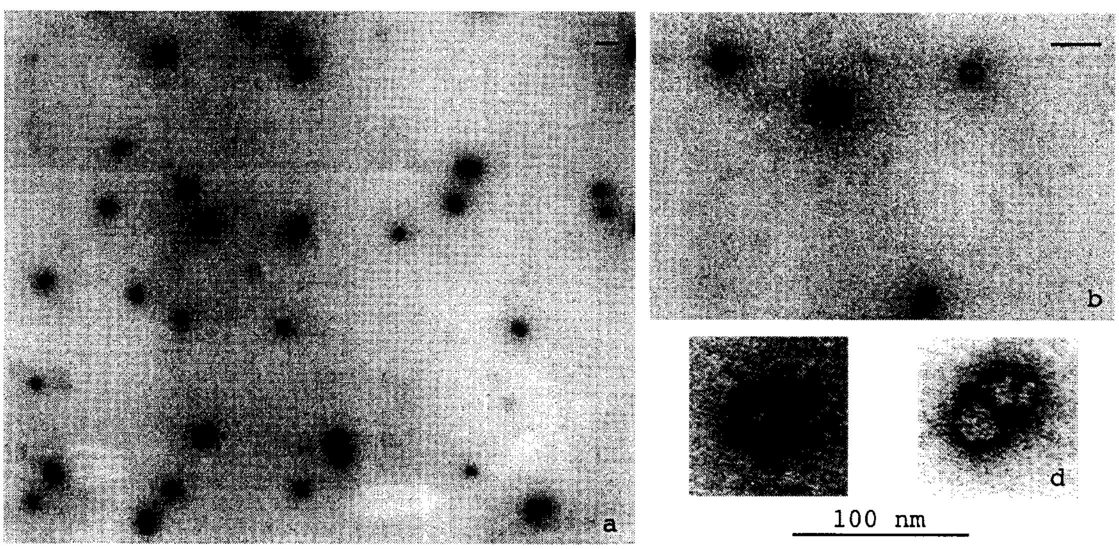

Fig. 5

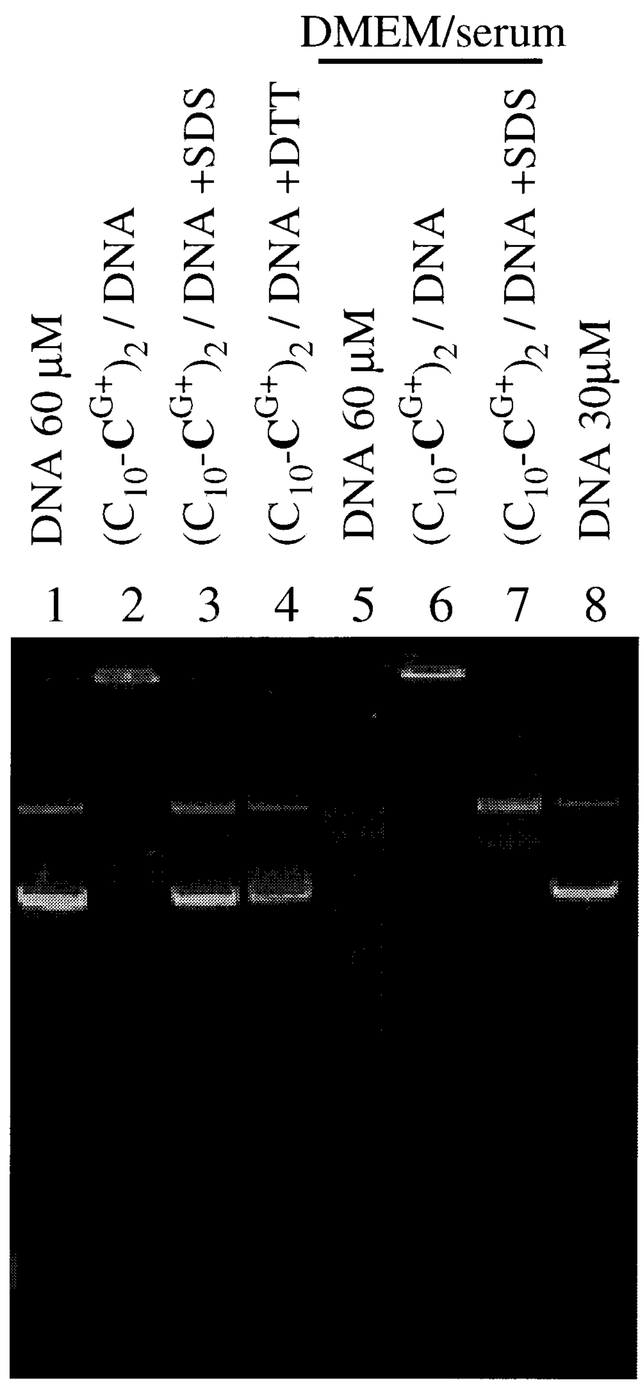
DMEM/serum
DNA 60 µM
(C10-CG+)2 / DNA
(C10-CG+)2 / DNA +SDS
(C10-CG+)2 / DNA +DTT
DNA 60 µM
(C10-CG+)2 / DNA
(C10-CG+)2 / DNA +SDS
DNA 30µM
1 2 3 4 5 6 7 8

Fig. 6A

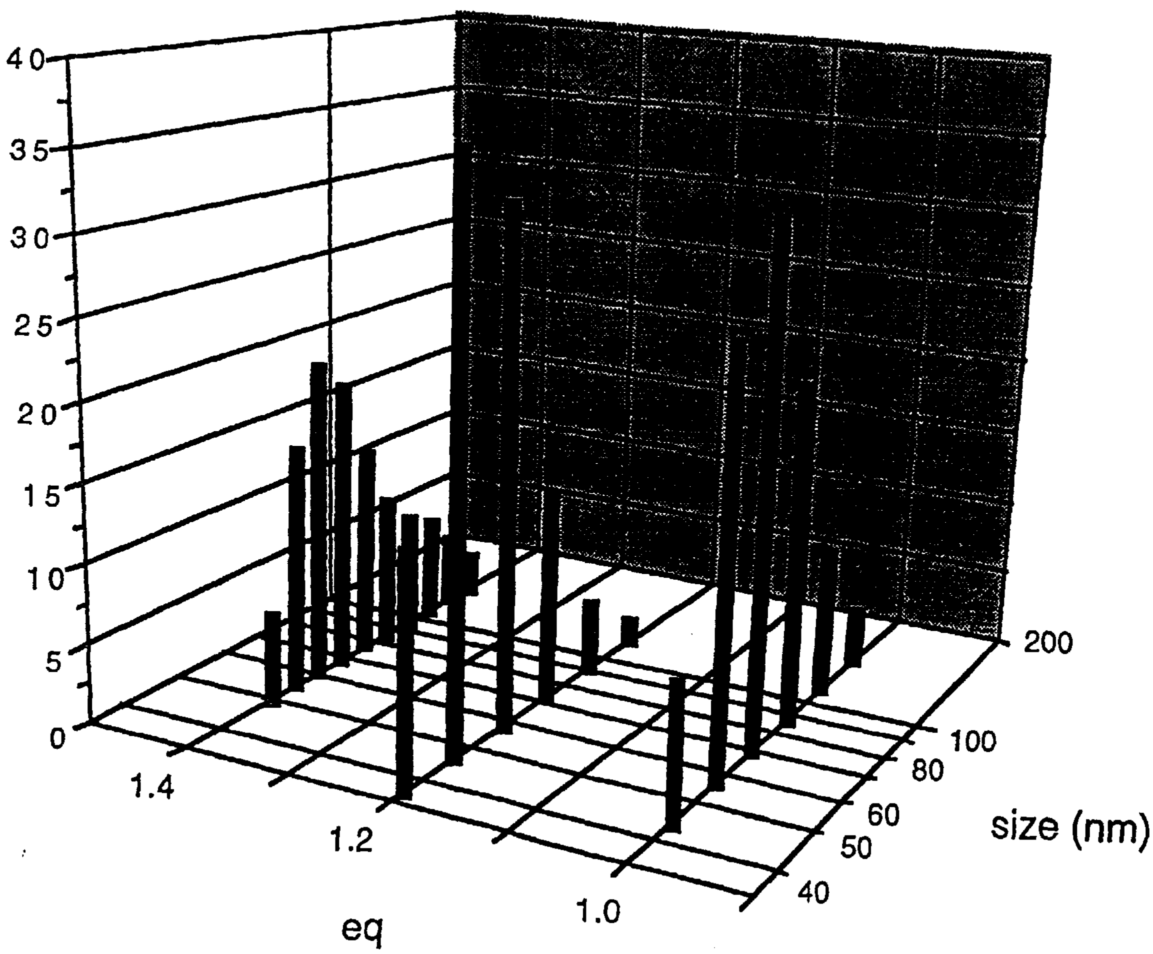

Fig. 6B

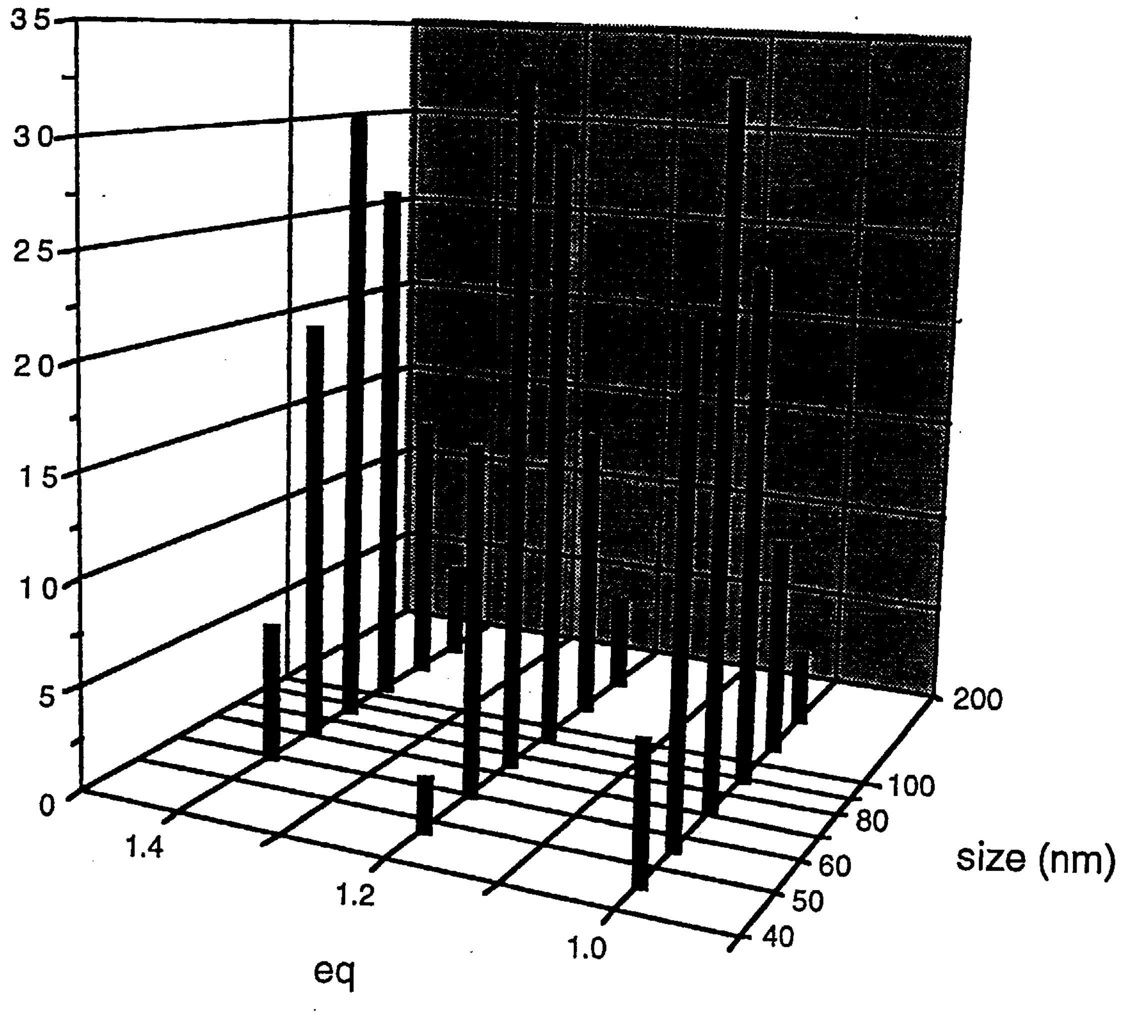

Fig. 6C

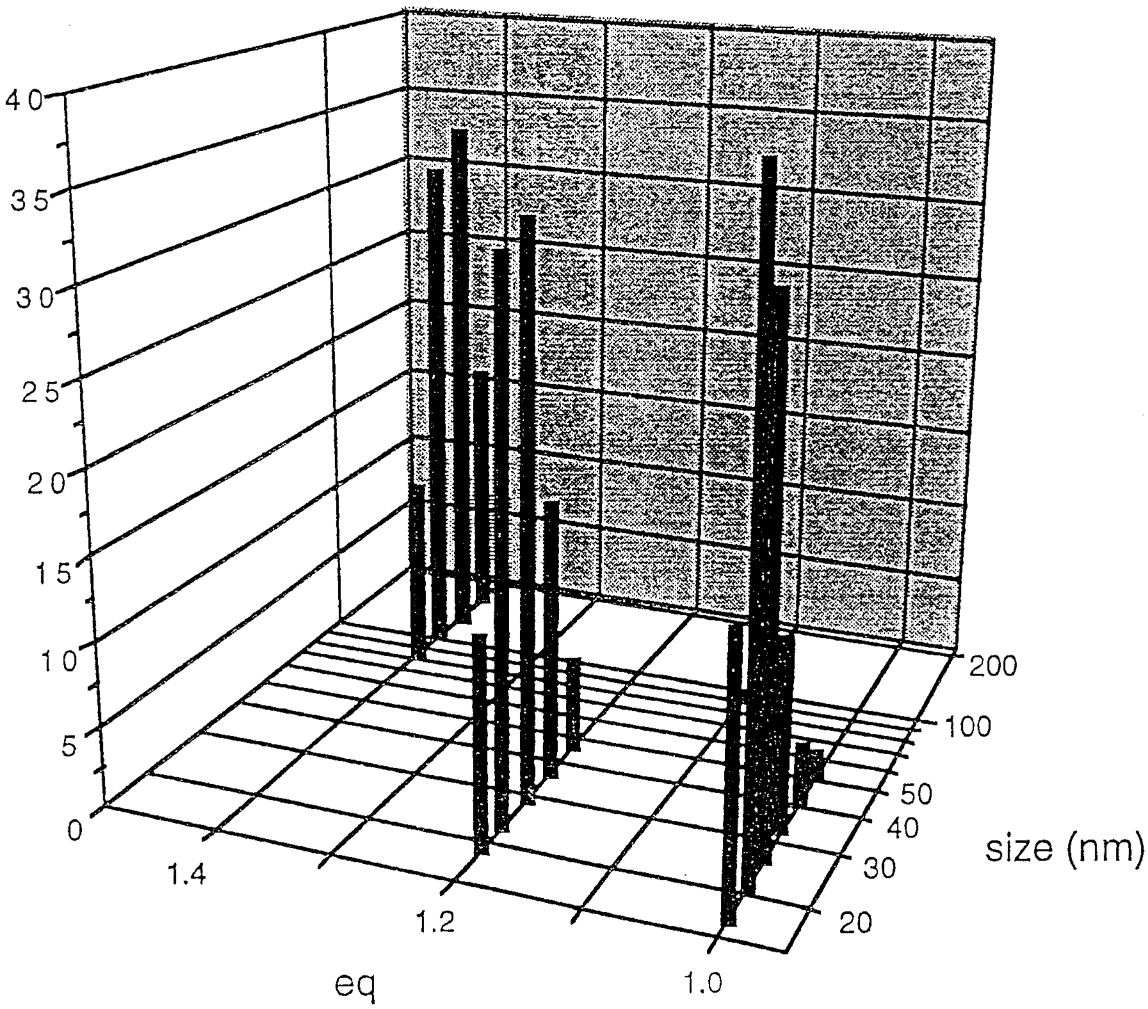

Fig. 7A

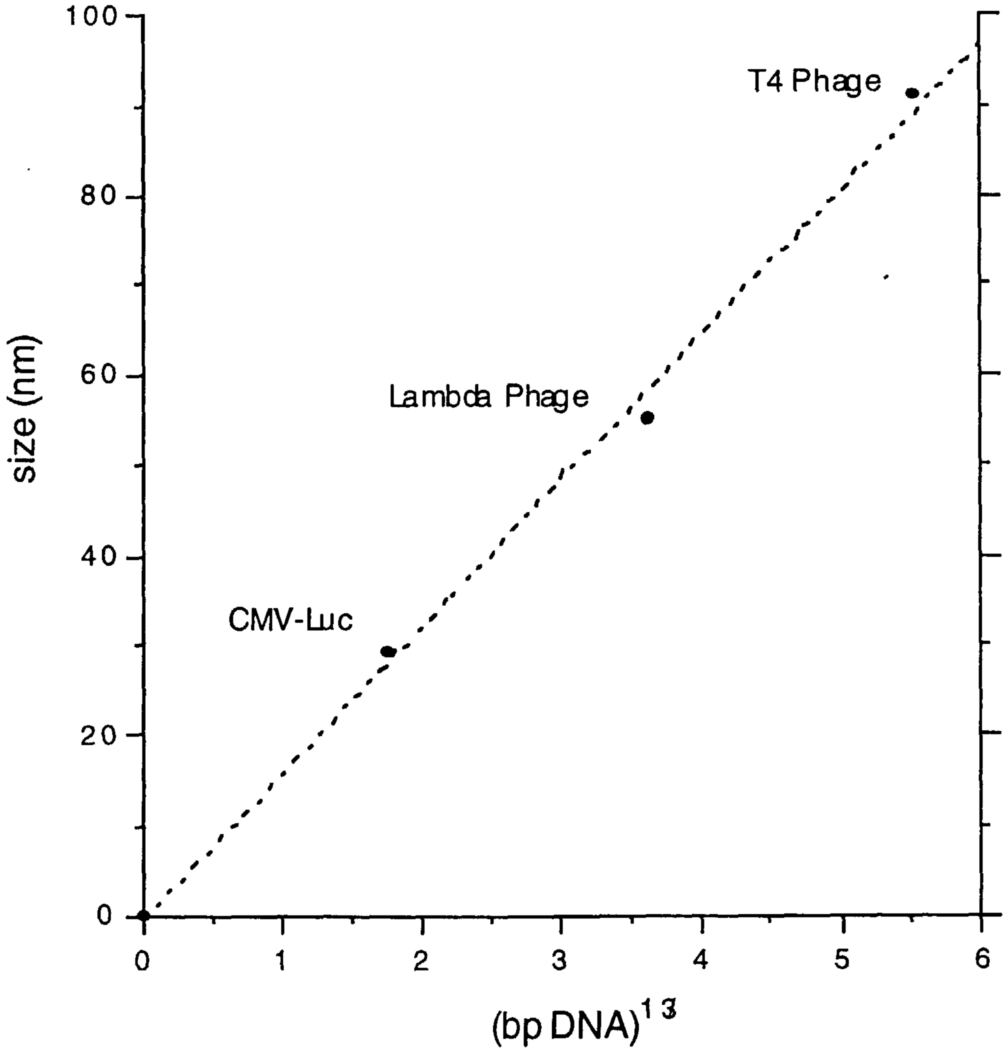
100
80
60
40
20
0
size (nm)
0
1
2
3
4
5
6
(bp DNA)1 3
T4 Phage
Lambda Phage
CMV-Luc

Fig. 7B

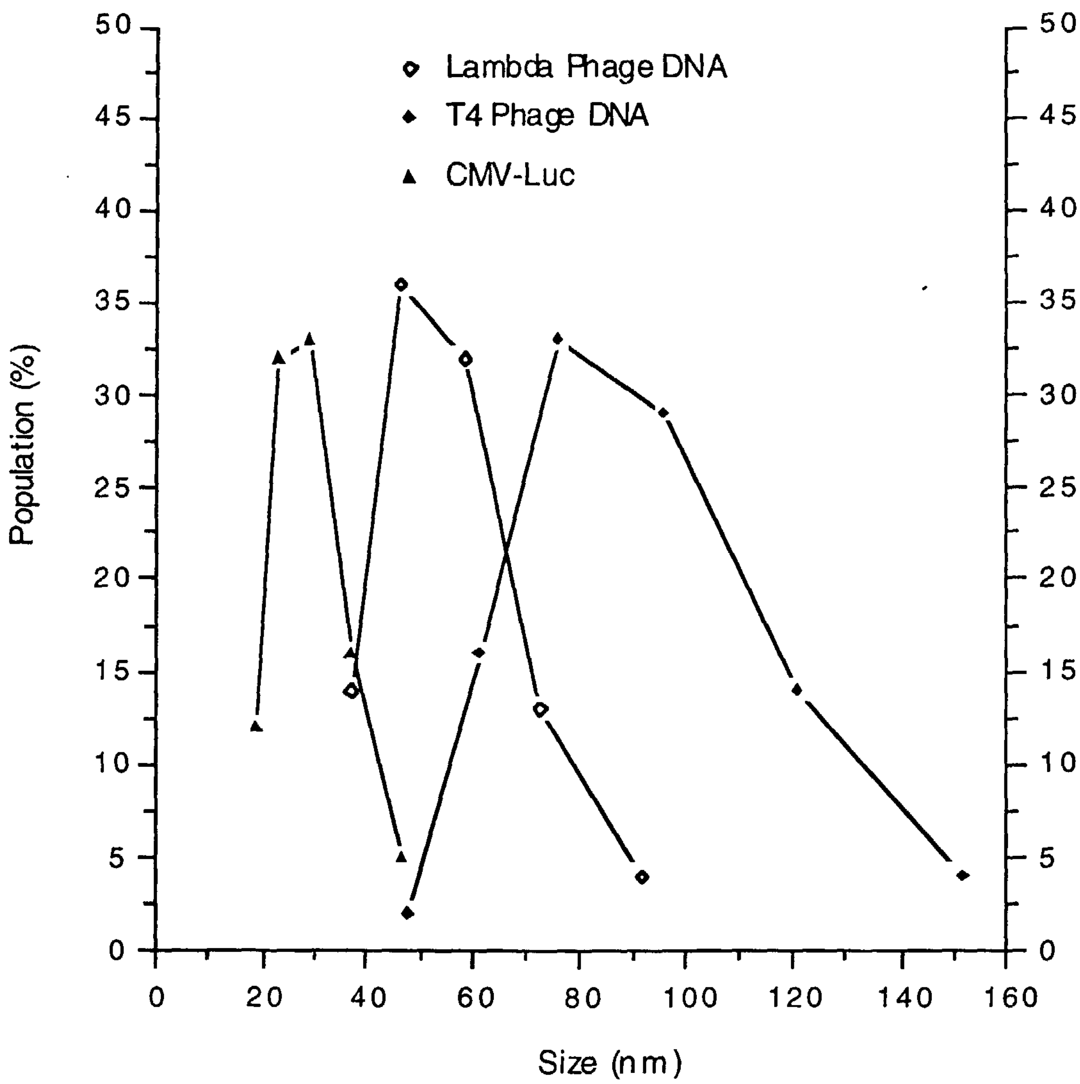
Lambda Phage DNA
T4 Phage DNA
CMV-Luc
Population (%)
Size (nm)
0
5
10
15
20
25
30
35
40
45
50
0
20
40
60
80
100
120
140
160

Fig. 8

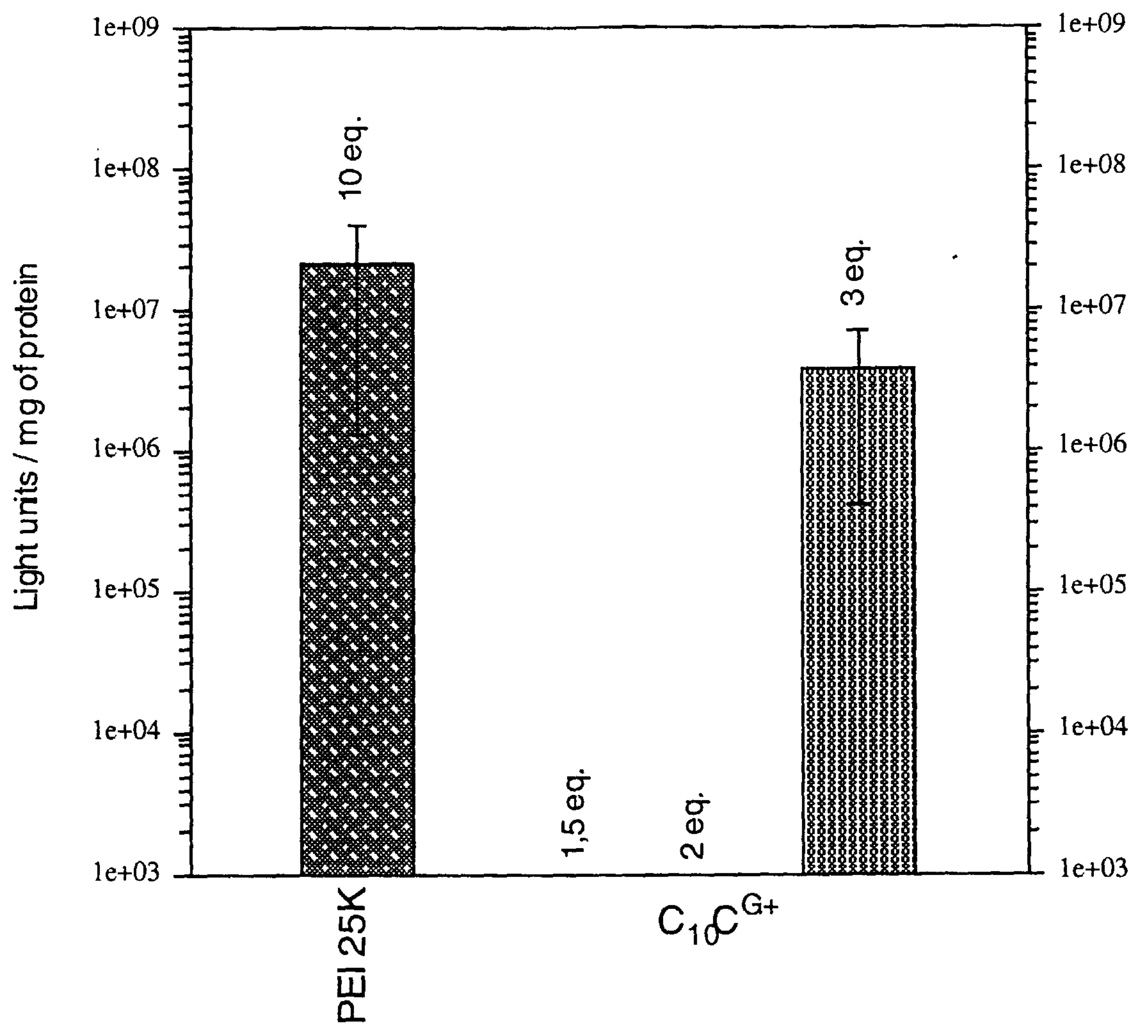
Light units / mg of protein
1e+09
1e+08
1e+07
1e+06
1e+05
1e+04
1e+03
10 eq.
1,5 eq.
2 eq.
3 eq.
PEI 25K
C10CG+

Fig. 9

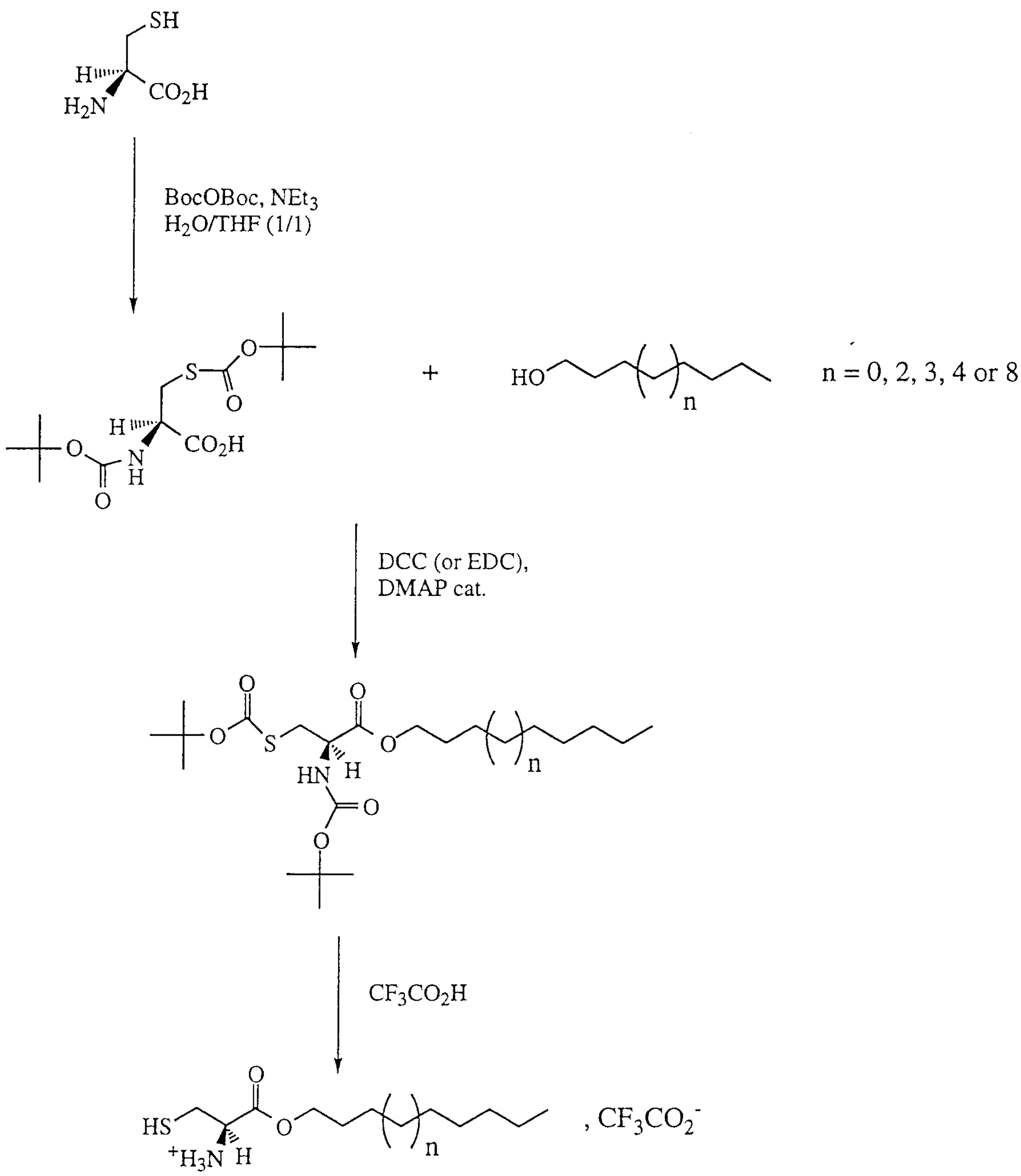
BocOBoc, NEt3
H2O/THF (1/1)
+
n = 0, 2, 3, 4 or 8
DCC (or EDC),
DMAP cat.
CF3CO2H
, CF3CO2-

Fig. 10

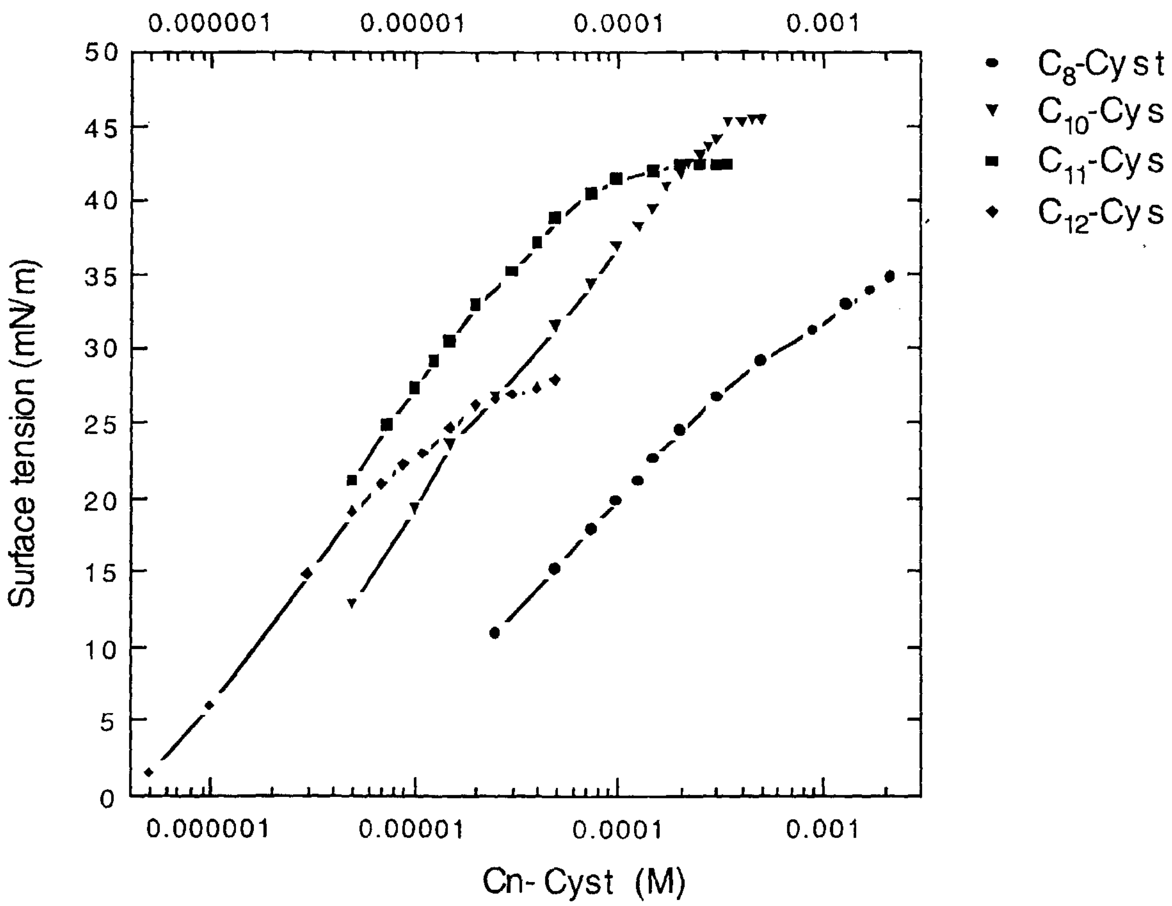
0.000001
0.00001
0.0001
0.001
50
45
40
35
30
25
20
15
10
5
0
Surface tension (mN/m)
Cn- Cyst (M)
C8-Cyst
C10-Cys
C11-Cys
C12-Cys

Fig. 11A

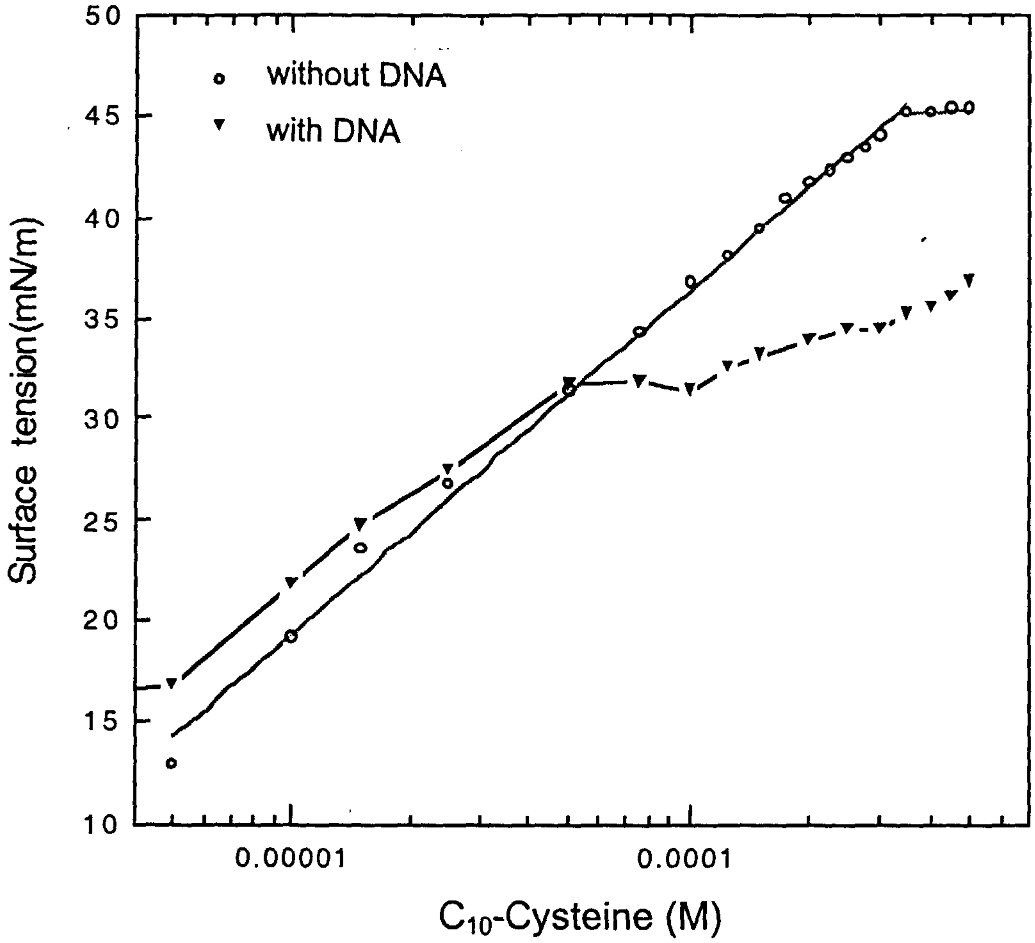
50
45
40
35
30
25
20
15
10
Surface tension(mN/m)
without DNA
with DNA
0.00001
0.0001
$C_{10}$-Cysteine (M)

Fig. 11B

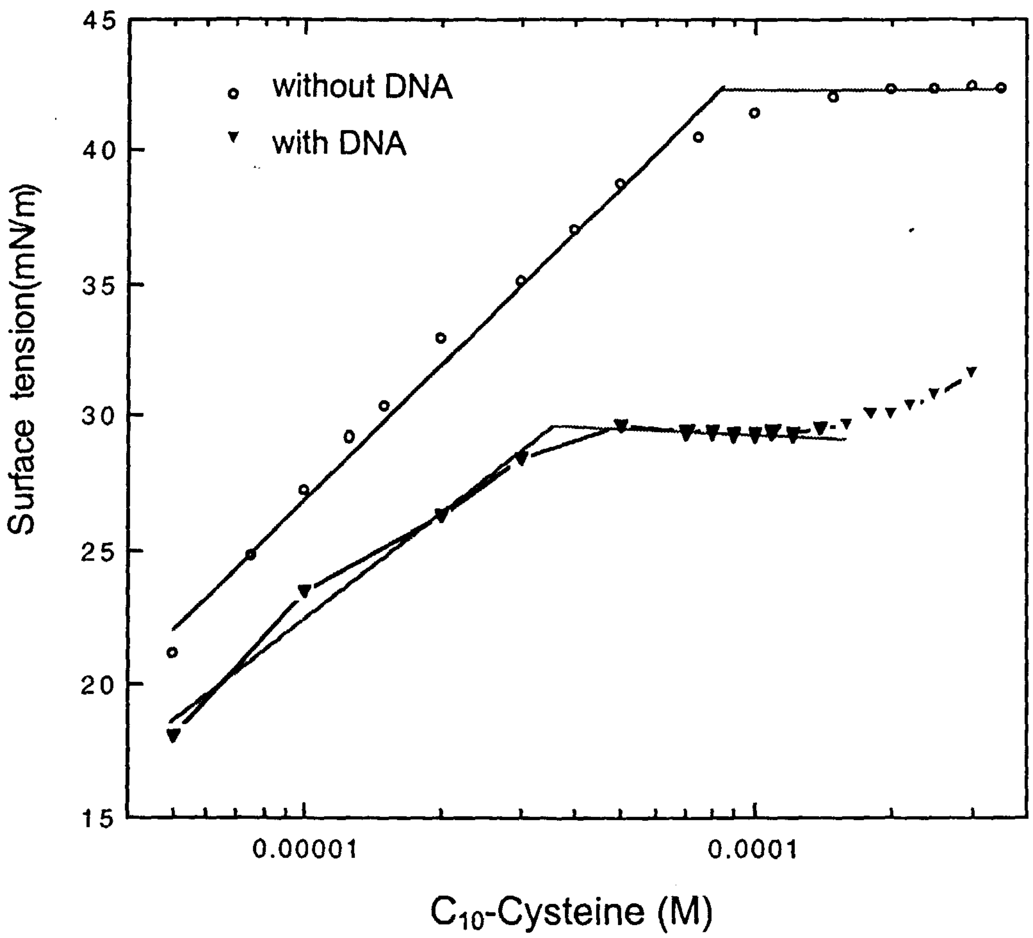
45
40
35
30
25
20
15
without DNA
with DNA
Surface tension(mN/m)
0.00001
0.0001
C10-Cysteine (M)

Fig. 12

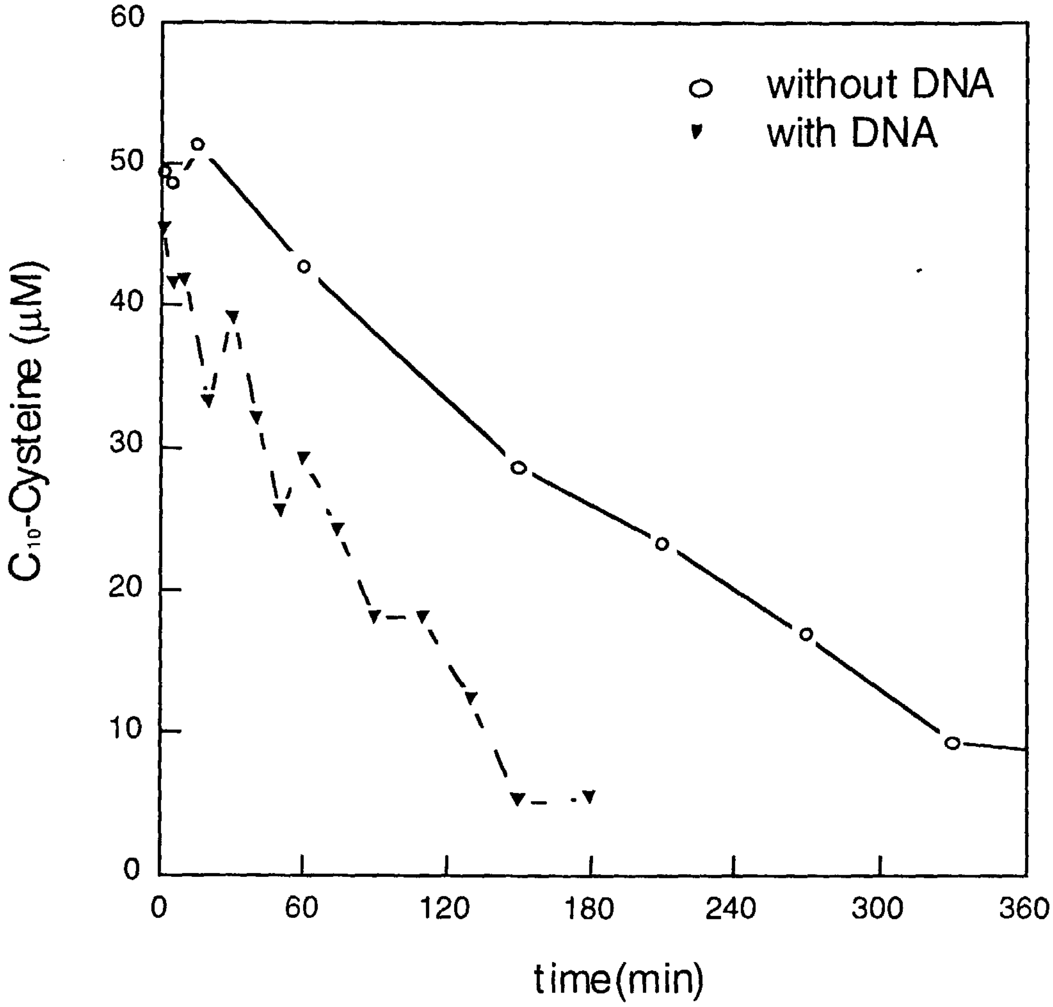

60
50
40
30
20
10
0
C10-Cysteine (μM)
0
60
120
180
240
300
360
time(min)
without DNA
with DNA

Fig. 13

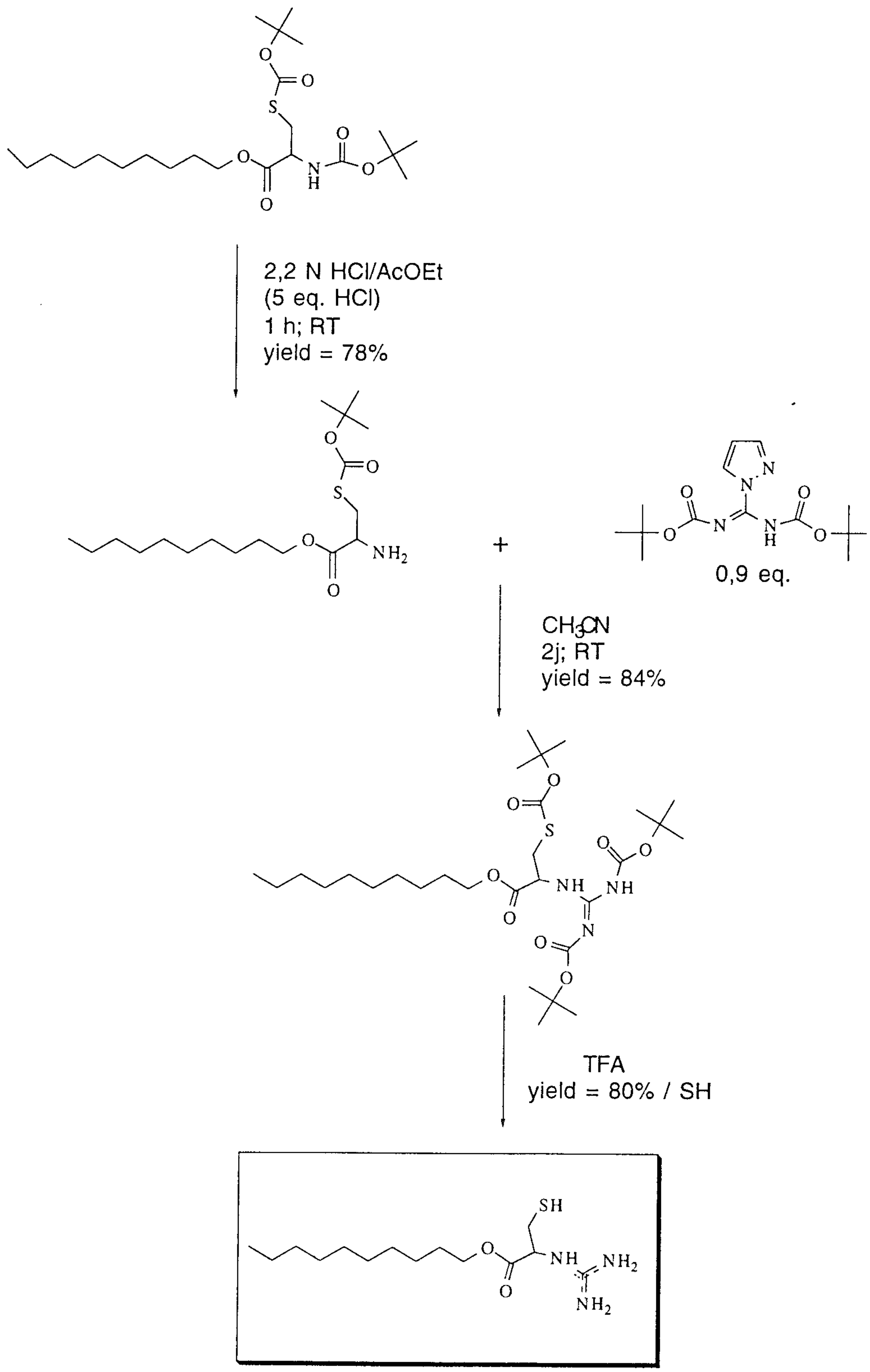
2,2 N HCl/AcOEt
(5 eq. HCl)
1 h; RT
yield = 78%
+
0,9 eq.
$CH_3CN$
2j; RT
yield = 84%
TFA
yield = 80% / SH

Fig. 14

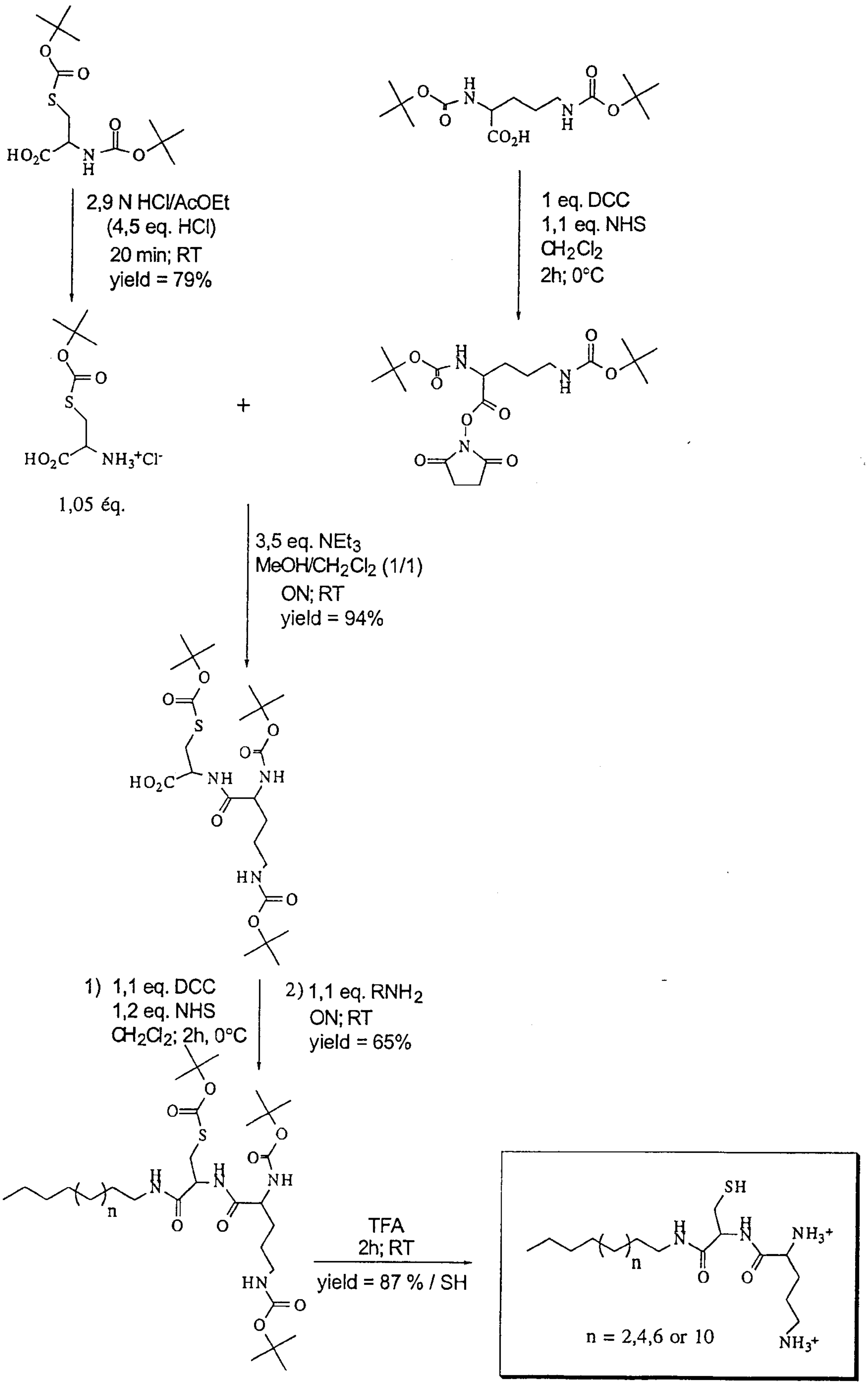
2,9 N HCl/AcOEt
(4,5 eq. HCl)
20 min; RT
yield = 79%
1 eq. DCC
1,1 eq. NHS
$CH_2Cl_2$
2h; 0°C
+
1,05 éq.
3,5 eq. $NEt_3$
MeOH/$CH_2Cl_2$ (1/1)
ON; RT
yield = 94%
1) 1,1 eq. DCC
1,2 eq. NHS
$CH_2Cl_2$; 2h, 0°C
2) 1,1 eq. $RNH_2$
ON; RT
yield = 65%
TFA
2h; RT
yield = 87 % / SH
n = 2,4,6 or 10

Fig. 15

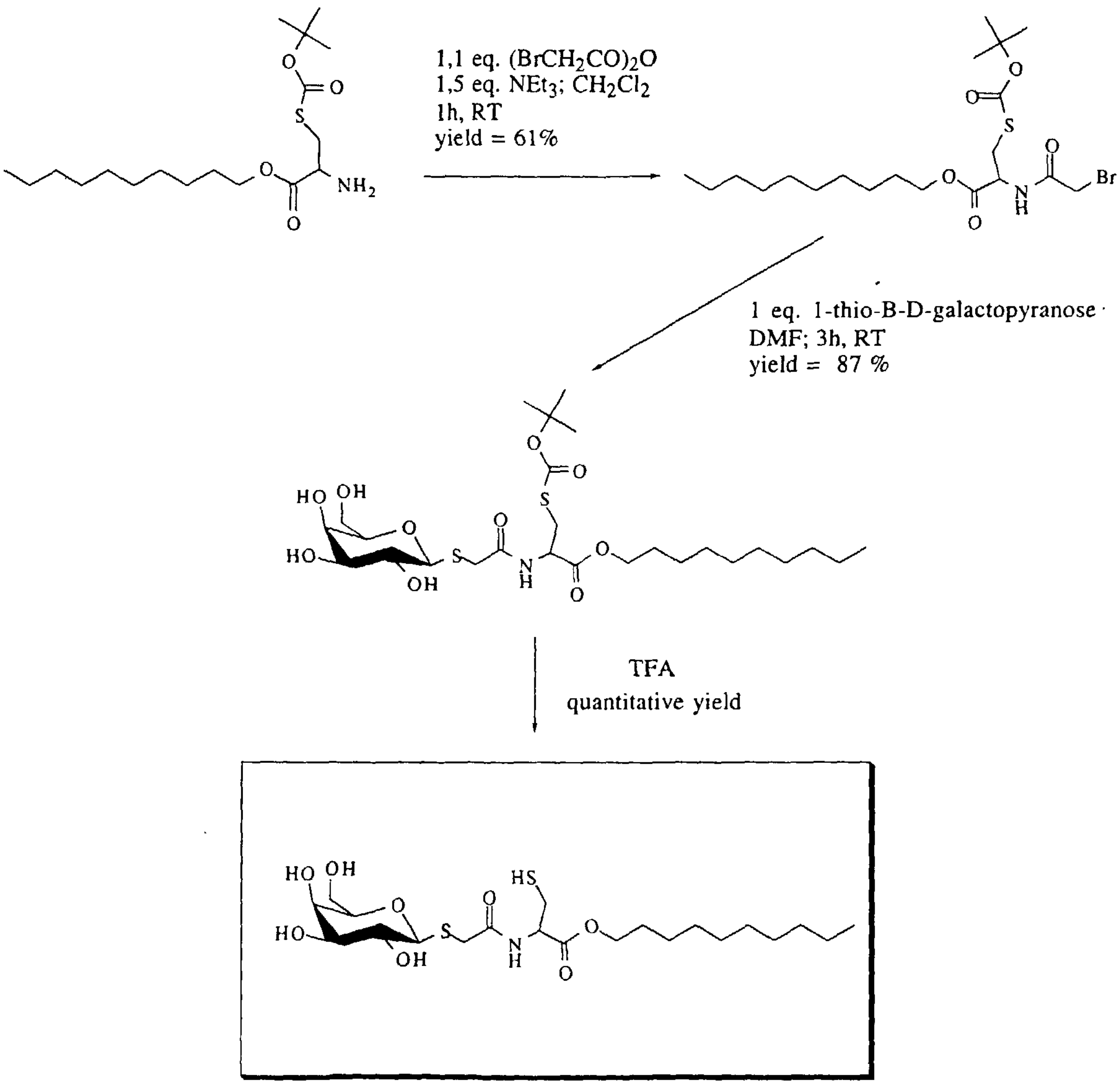
1,1 eq. $(BrCH_2CO)_2O$
1,5 eq. $NEt_3$; $CH_2Cl_2$
1h, RT
yield = 61%
1 eq. 1-thio-B-D-galactopyranose
DMF; 3h, RT
yield = 87 %
TFA
quantitative yield

**Fig. 16A**

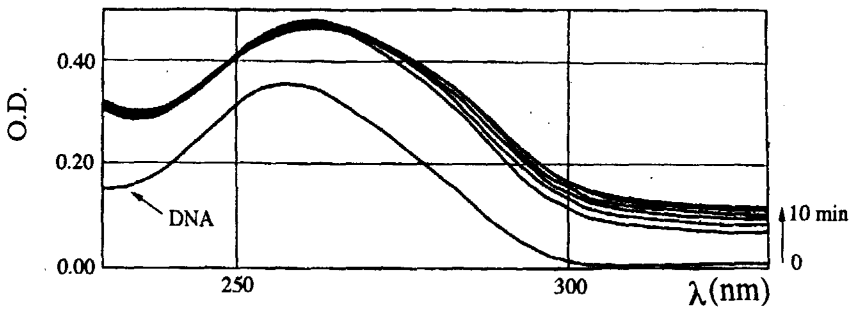


**Fig. 16B**

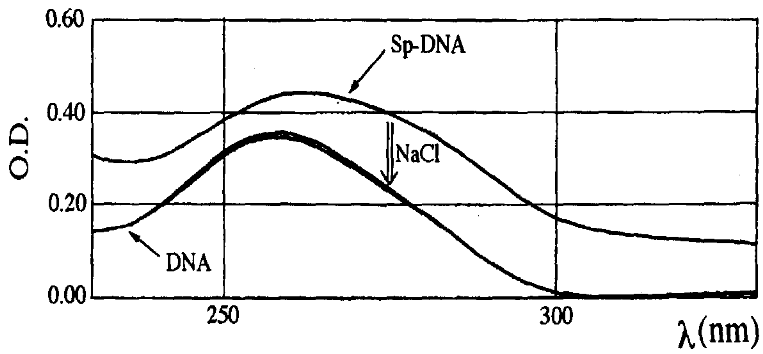


**Fig. 16C**

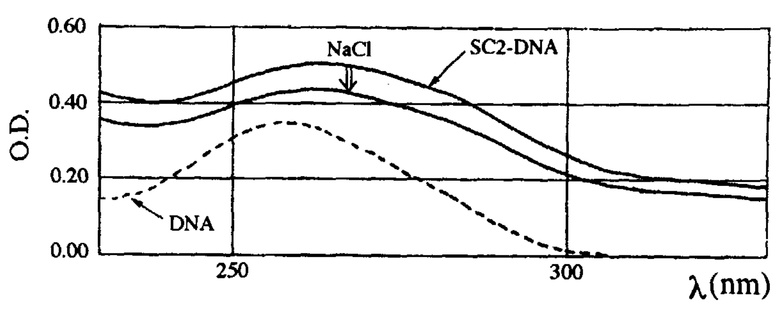

Fig. 17

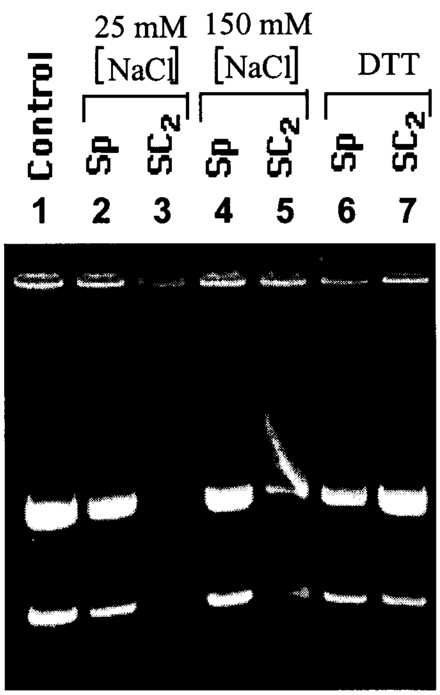

European Patent Office

## PARTIAL EUROPEAN SEARCH REPORT

which under Rule 45 of the European Patent Convention shall be considered, for the purposes of subsequent proceedings, as the European search report

Application Number EP 97 12 1308

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | BLESSING, THOMAS ET AL: "Monomolecular collapse of plasmid DNA into stable virus-like particles" PROC. NATL. ACAD. SCI. U. S. A. (1998), 95(4), 1427-1431 CODEN: PNASA6;ISSN: 0027-8424, 1998, XP002066309 * the whole document * --- | 1-39 | A61K47/48 |
| Y | REIMER, DOROTHY L. ET AL: "Formation of Novel Hydrophobic Complexes between Cationic Lipids and Plasmid DNA" BIOCHEMISTRY (1995), 34(39), 12877-83 CODEN: BICHAW;ISSN: 0006-2960, 1995, XP002066310 * page 12882, column 1, paragraph 3; figure 7 * --- | 1-39 | |
| A | US 4 873 187 A (TAUB FLOYD) 10 October 1989 * claim 10 * --- | 1-39 | TECHNICAL FIELDS SEARCHED (Int.Cl.6) |
| | -/-- | | A61K |

### INCOMPLETE SEARCH

The Search Division considers that the present application, or some or all of its claims, does/do not comply with the EPC to such an extent that a meaningful search into the state of the art cannot be carried out, or can only be carried out partially, for the following claims:

Claims searched completely :

Claims searched incompletely :

Claims not searched :

Reason for the limitation of the search:

see sheet C

| Place of search | Date of completion of the search | Examiner |
|---|---|---|
| THE HAGUE | 28 May 1998 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 97 12 1308

| DOCUMENTS CONSIDERED TO BE RELEVANT | | | CLASSIFICATION OF THE APPLICATION (Int.Cl.6) |
|---|---|---|---|
| Category | Citation of document with indication, where appropriate, of relevant passages | Relevant to claim | |
| P,X | WO 98 10649 A (UNIV TECHNOLOGY CORP) 19 March 1998<br>* claims 1,9,27,34,40,41,44 *<br>--- | 1 | |
| Y | GERSHON, HEZI ET AL: "Mode of formation and structural features of DNA -cationic liposome complexes used for transfection" BIOCHEMISTRY (1993), 32(28), 7143-51 CODEN: BICHAW;ISSN: 0006-2960, 1993, XP000601477<br>* abstract *<br>--- | 1-39 | |
| Y | WO 93 07283 A (BOEHRINGER INGELHEIM INT ;UNIV NORTH CAROLINA (US); GENENTECH INC) 15 April 1993<br>* claims *<br>----- | 1-39 | TECHNICAL FIELDS SEARCHED (Int.Cl.6) |

EPO FORM 1503 03.82 (P04C10)

European Patent Office

**INCOMPLETE SEARCH SHEET C**

Application Number
EP 97 12 1308

Claim(s) searched incompletely:
1-39

Reason for the limitation of the search:

Reason for the limitation of the search: In view of the large number of compounds which are designed by the compounds mentioned in the claims, The search was limited to the compounds for which pharmacological data was given and/or
the compounds mentioned in the claims or examples.